(19) Europäisches Patentamt
European Patent Office
Office européen des brevets

(11) **EP 4 620 495 A1**

(12) **EUROPEAN PATENT APPLICATION**

(43) Date of publication:
**24.09.2025 Bulletin 2025/39**

(21) Application number: **24165486.2**

(22) Date of filing: **22.03.2024**

(51) International Patent Classification (IPC):
***A61L 27/56*** *(2006.01)* ***A61L 27/48*** *(2006.01)*

(52) Cooperative Patent Classification (CPC):
(C-Sets available)
**A61L 27/56; A61L 27/48;** A61L 2430/02;
A61L 2430/12 (Cont.)

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(71) Applicant: **Université de Liège**
**4000 Liège (BE)**

(72) Inventors:
- **JEROME, Christine**
  **4102 Ougrée (BE)**
- **TAGHIPOUR, Hamid**
  **4020 Liège (BE)**
- **VAN HEDE, Dorien**
  **4550 Nandrin (BE)**
- **RIVA, Raphaël**
  **4400 Flémalle (BE)**
- **LAMBERT, France**
  **4870 Trooz (BE)**

(74) Representative: **Winger**
**Mouterij 16 bus 101**
**3190 Boortmeerbeek (BE)**

(54) **DEGRADABLE FIBRE BLEND FOR BONE REGENERATION**

(57) The present invention relates to a fibre blend for bone regeneration comprising at least a degradable polyester fibre and a degradable copolymer fibre, said copolymer fibre comprising degradable ester units and from 1 to 50 % in number of phosphoester units versus the total number of monomer units in said copolymer fibre, as well as to a process for preparing a fibre blend and a membrane comprising the fibre blend.

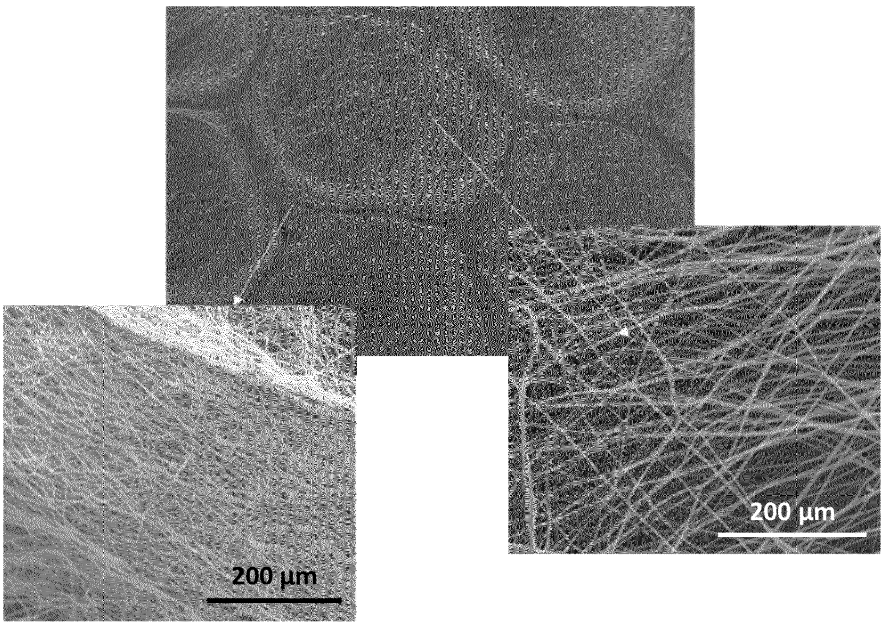


Figure 13

EP 4 620 495 A1

(52) Cooperative Patent Classification (CPC): (Cont.)

C-Sets
**A61L 27/48, C08L 67/04;**
**A61L 27/48, C08L 85/02**

## Description

### Technical field of the invention

The present invention relates to the field of degradable fibre blends for bone regeneration, to processes for the preparation of such fibre blends, to resorbable membranes for bone regeneration and their use in dental implants.

### Background of the invention

Several million dental implants are placed each year worldwide. Among these, approximately 30% require prior bone regeneration. The principle of bone regeneration is the creation of a volume between the residual bone surface and the muco-periosteal flap, this volume being filled with a filling material, for example hydroxyapatite, and surrounded by a membrane which plays the role of a barrier between the soft tissues and the space to be regenerated. This membrane is essential in preventing the migration of the soft tissues into the space to be regenerated so as to allow the more slowly growing bone tissue to colonize the volume created.

There are currently two types of membranes: resorbable and non-resorbable membranes. The latter, most often made of Gore-Tex® and therefore biologically inert, are appreciated for their mechanical strength once in place. However, these need to be fixed with screws and to be removed during a second surgery, which significantly increases the morbidity of the procedure. In addition, their use is associated with an increased risk of postoperative complications. The second type, resorbable membranes, are resorbed after a period dependent from their composition and therefore do not require an additional surgical procedure. However, their rate of resorption must be controlled and their degradation products must be biocompatible, i.e. not induce excessive inflammation. Moreover, resorbable membranes often lack mechanical performance to compete with non-resorbable membranes.

There is therefore a need for membranes overcoming at least partially one or more of the issues mentioned above.

### Summary **of the invention**

It is an object of the present invention to provide a fibre blend which is suitable for use as dental implants.

The above objective is accomplished by a fibre blend which presents good mechanical properties and is biocompatible, as well as by a process for the preparation of such fibre blend and a membrane comprising such fibre blend.

It is an advantage of embodiments of the present invention that the fibre blend according to the invention has a high mechanical strength. It is also an advantage of embodiments of the present invention that it presents a good balance between rigidity and softness. The mechanical properties are advantageously maintained for about three to six months, after which resorption starts. Other advantages of the fibre blend according to embodiments of the invention are an increased hydrophilicity, modular surface properties and a high porous volume.

In a first aspect, the present invention relates to a fibre blend for bone regeneration comprising at least a degradable polyester fibre and a degradable copolymer fibre, said copolymer fibre comprising degradable ester units and from 1 to 50 % in number of phosphoester units versus the total number of monomer units in said copolymer fibre.

In a second aspect, the present invention relates to a process for preparing a fibre blend comprising the steps of:

(i) providing a solution of a degradable polyester and a degradable copolymer, said copolymer comprising degradable ester units and from 1 to 50 % in number of phosphoester units versus the total number of monomer units in said copolymer, then
(ii) electrospinning said solution so as to obtain a fibre blend, then
(iii) optionally releasing negative charges of phosphoester units of the fibre blend, then
(iv) optionally dipping the fibre blend in a polysaccharide solution so as to obtain a coated fibre blend, then
(v) optionally dipping the coated fibre blend in a poly(phosphoester) solution and subsequently dipping the fibre blend in a polysaccharide solution so as to obtain a layered coated fibre blend, and then
(vi) optionally repeating step (v) until the desired layer number is reached.

In a third aspect, the present invention relates to a membrane comprising the fibre blend according to the first aspect wherein said membrane has a thickness of from 1 micrometer to 1 millimeter, preferably from 50 micrometers to 500 micrometers, more preferably from 100 micrometers to 300 micrometers.

In a fourth aspect, the present invention relates to a fibre blend according to the first aspect or a membrane according to the third aspect for use in a medical treatment.

Particular and preferred aspects of the invention are set out in the accompanying independent and dependent claims. Features from the dependent claims may be combined with features of the independent claims and with features of

other dependent claims as appropriate and not merely as explicitly set out in the claims.

The above and other characteristics, features, and advantages of the present invention will become apparent from the following detailed description, taken in conjunction with the accompanying drawings, which illustrate, by way of example, the principles of the invention. This description is given for the sake of example only, without limiting the scope of the invention. The reference figures quoted below refer to the attached drawings.

## Brief description of the drawings

Figure 1 shows contact angle measurement on PCL fibre mat (a) and on PCL/PCL-b-PPE fibre blend mat (b).

Figure 2 shows stress-strain curves of PCL fibre (A), of fibre blend PCL/PCL-b-PPE (B) and of fibre blend coated by chitosan (PCL/PCL-b-PPE/CS) (C).

Figure 3 shows the evolution of the Young modulus after an ageing of 6 months in triplicate for sample A and sample B.

Figure 4 shows the evolution of the failure in % after an ageing of 6 months in triplicate for sample A and sample B.

Figure 5 shows SEM images of fibre blend PCL/PCL-b-PPE after chitosan coating (left) and before chitosan coating (right).

Figure 6 shows SEM images at higher magnification of PCL/PCL-b-PPE after 5 layers of chitosan coating (left) and before chitosan coating (right).

Figure 7 shows an image of the section of fibre blend PCL/PCL-b-PPE after 5 layers of chitosan coating, recorded with a fluorescence microscope at 480 nm. Lighter part corresponds to fluorescent chitosan.

Figure 8 shows a graph of zeta potential of PCL fibre and of fibre blends according to various embodiments of the invention.

Figure 9 show XPS analysis (full spectra) for electrospun membranes of PCL fibre (top), fibre blend PCL/PCL-b-PPE (middle) and fibre blend PCL/PCL-b-PPE after deprotection of negative charges (bottom).

Figure 10 show areas C1s, O1s and P2p of XPS analysis for electrospun membranes of PCL fibre (top), fibre blend PCL/PCL-b-PPE (middle) and fibre blend PCL/PCL-b-PPE after deprotection of negative charges (bottom).

Figure 11 show XPS analysis (full spectra) for electrospun membranes after chitosan coating of PCL fibre (top), fibre blend PCL/PCL-*b*-PPE (middle) and fibre blend PCL/PCL-*b*-PPE after deprotection of negative charges (bottom).

Figure 12 show areas C1s and O1s (A), P2p and N1s (B) of XPS analysis for electrospun membranes after chitosan coating of PCL fibre (top), fibre blend PCL/PCL-*b*-PPE (middle) and fibre blend PCL/PCL-*b*-PPE after deprotection of negative charges (bottom).

Figure 13 shows SEM images of a structured membrane of a fibre blend according one embodiment of the invention.

## Description of illustrative embodiments

The present invention will be described with respect to particular embodiments and with reference to certain drawings but the invention is not limited thereto but only by the claims. The drawings described are only schematic and are non-limiting. In the drawings, the size of some of the elements may be exaggerated and not drawn on scale for illustrative purposes. The dimensions and the relative dimensions do not correspond to actual reductions to practice of the invention.

Furthermore, the terms first, second, third, and the like in the description and in the claims, are used for distinguishing between similar elements and not necessarily for describing a sequence, either temporally, spatially, in ranking, or in any other manner. It is to be understood that the terms so used are interchangeable under appropriate circumstances and that the embodiments of the invention described herein are capable of operation in other sequences than described or illustrated herein.

Moreover, the terms top, bottom, over, under and the like in the description and the claims are used for descriptive purposes and not necessarily for describing relative positions. It is to be understood that the terms so used are interchangeable under appropriate circumstances and that the embodiments of the invention described herein are capable of operation in other orientations than described or illustrated herein.

The terms "over" and "above" are used as synonyms and cover situations with and without physical contacts. The term "on" means "over and in physical contact with".

It is to be noticed that the term "comprising", used in the claims, should not be interpreted as being restricted to the means listed thereafter; it does not exclude other elements or steps. It is thus to be interpreted as specifying the presence of the stated features, integers, steps, or components as referred to, but does not preclude the presence or addition of one or more other features, integers, steps or components, or groups thereof. The term "comprising" therefore covers the situation where only the stated features are present (and can therefore always be replaced by "consisting of" in order to restrict the scope to said stated features) and the situation where these features and one or more other features are present. The word "comprising" according to the invention therefore also includes as one embodiment that no further

components are present. Thus, the scope of the expression "a device comprising means A and B" should not be interpreted as being limited to devices consisting only of components A and B. It means that with respect to the present invention, the only relevant components of the device are A and B.

Reference throughout this specification to "one embodiment" or "an embodiment" means that a particular feature, structure, or characteristic described in connection with the embodiment is included in at least one embodiment of the present invention. Thus, appearances of the phrases "in one embodiment" or "in an embodiment" in various places throughout this specification are not necessarily all referring to the same embodiment, but may. Furthermore, the particular features, structures, or characteristics may be combined in any suitable manner, as would be apparent to one of ordinary skill in the art from this disclosure, in one or more embodiments.

Similarly, it should be appreciated that in the description of exemplary embodiments of the invention, various features of the invention are sometimes grouped together in a single embodiment, figure, or description thereof for the purpose of streamlining the disclosure and aiding in the understanding of one or more of the various inventive aspects. This method of disclosure, however, is not to be interpreted as reflecting an intention that the claimed invention requires more features than are expressly recited in each claim. Rather, as the following claims reflect, inventive aspects lie in less than all features of a single foregoing disclosed embodiment. Thus, the claims following the detailed description are hereby expressly incorporated into this detailed description, with each claim standing on its own as a separate embodiment of this invention.

Furthermore, while some embodiments described herein include some but not other features included in other embodiments, combinations of features of different embodiments are meant to be within the scope of the invention, and form different embodiments, as would be understood by those in the art. For example, in the following claims, any of the claimed embodiments can be used in any combination.

Furthermore, some of the embodiments are described herein as a method or combination of elements of a method that can be implemented by a processor of a computer system or by other means of carrying out the function. Thus, a processor with the necessary instructions for carrying out such a method or element of a method forms a means for carrying out the method or element of a method. Furthermore, an element described herein of an apparatus embodiment is an example of a means for carrying out the function performed by the element for the purpose of carrying out the invention.

In the description provided herein, numerous specific details are set forth. However, it is understood that embodiments of the invention may be practiced without these specific details. In other instances, well-known methods, structures, and techniques have not been shown in detail in order not to obscure an understanding of this description.

The following terms are provided solely to aid in the understanding of the invention.

In the context of the invention, a blend refers to a composition of at least two compounds having different properties. It particularly refers to a composition of two polymers having different properties. The term blend is particularly used in the field of fibres and/or electrospinning, during which fibres are projected from a solution comprising two compounds. After such electrospinning fibres of one compound and of the at least second compounds are mixed. A blend thus refers to a mixture of two or more fibres.

In the context of the invention, bone regeneration refers to the principle of creation of a volume between a residual bone surface and a section of soft tissue, such as for example the muco-periosteal flap, this volume being filled with a filling material, for example hydroxyapatite, and surrounded by a membrane which plays the role of a barrier between the soft tissues and the space to be regenerated.

In the context of the invention, degradable refers to the property to be resorbable in a human or animal body.

In the context of the invention, a copolymer refers to a polymer formed from the polymerization of at least two monomer units differing in chemical nature. Monomer units may be randomly distributed (also referred to as statistically distributed) or have a gradient distribution or be sequentially distributed, the later leading to block copolymers. Monomer units, or simply units, are repetitive units of polymers. Any polymer or copolymer is formed by the assembly of monomer units.

In the context of the invention, a block copolymer refers to a polymer formed of at least two polymers, differing in chemical nature, and attached (e.g., covalently) to each other. Each polymer composing the block copolymer is called a block or a chain. Each block can either be a homopolymer or a copolymer selected from statistical copolymers and gradient copolymers.

A random distribution of monomer units is obtained when two or more monomer units are in presence during the polymerization step. A block copolymerization is obtained when two of more monomer units are sequentially added during the polymerization step.

By an alkyl group, one means a monovalent straight or branched, unsubstituted and saturated hydrocarbyl group, for example a methyl, an ethyl, a propyl, a butyl, an isobutyl, a hexyl and the like. By substituted alkyl, on the other hand, one means an alkyl group in which one or more hydrogen atoms of the alkyl chain are replaced by functional groups containing one or more heteroatoms. Examples of substituted alkyls comprise hydroxyalkyls (e.g., 2-hydroxyethyl), aminoalkyls (e.g., 2-aminopropyl), haloalkyls (e.g., chloromethyl or 2-bromoethyl), and alkoxyalkyls (e.g., methoxyethyl or ethoxyethyl), alkylthioalkyls (e.g., ethylthiomethyl), amongst others.

By an alkylene group, one means a divalent straight or branched, unsubstituted and saturated hydrocarbyl skeleton that acts as a bridge between two molecular entities. Examples are methylene ($-CH_2-$), ethylene ($-CH_2CH_2-$), propylene ($-CH_2CH_2CH_2-$), butylene ($-CH_2CH_2CH_2CH_2-$), and similar configurations. By substituted alkylene, one refers to an alkylene group in which one or more hydrogen atoms of the alkylene chain are replaced by functional groups containing one or more heteroatoms. Examples are hydroxyalkylene (e.g., $-CH_2-CH(OH)-$), aminoalkylene (e.g., $-CH_2-CH_2-CH(NH_2)-$), haloalkylene (e.g., -CHCl-), alkoxyalkylene (e.g., $-CH-CH(OCH_3)-$), and alkylthioalkylene (e.g., $-CH(SCH_2CH_3)-$), among others.

By an alkoxy group, one means a monovalent group of the formula -OR, where R is an alkyl group as defined above. Examples include methoxy ($-OCH_3$), ethoxy ($-OCH_2CH_3$), propoxy ($-OCH_2CH_2CH_3$), and the like.

By mechanical properties, one means one of stiffness, softness, elongation at break, Young's modulus, stretchability, elasticity. Mechanical properties are measured in dry state and/or in wet state.

According to a first aspect, the invention relates to a fibre blend for bone regeneration comprising at least a degradable polyester fibre and a degradable copolymer fibre, said copolymer fibre comprising degradable ester units and from 1 to 50 % in number of phosphoester units versus the total number of monomer units in said copolymer fibre.

It is an advantage of embodiments of the present invention that such a fibre blend present good mechanical properties, thanks to the presence of the polyester fibre. In particular embodiments, it is advantageous that the fibre blend presents a high mechanical strength, such as a mechanical strength which enables to form a membrane that is easy to manipulate, in particular that is easy to be cut by suitable tools and/or by an ordinary practitioner. The mechanical strength measured in dry state may for example be between 1 and 20 MPa. Furthermore, in embodiments, the fibre blend presents a good balance between its rigidity and its softness. Advantageously this can be modulated by the dry or wet environment in which the fibre blend is placed. For example, the softness may be increased when placed in a wet environment, preferably an aqueous environment. By good mechanical properties, it is further understood that the properties are maintained for a certain duration before the start of degradation. For example, the fibre blend according to the invention only starts to be degraded after a few months, such as after 3 to 6 months.

Degradable polyester fibres are fibres made of degradable polyester. Degradable polyester is preferably selected from the list consisting of polylactide (PLA), polylactic-co-glycolic acid (PLGA), polyglycolic acid (PGA), polycaprolactone (PCL), polyanhydride, polyhydroxyalcanoate (PHA), isomers thereof, copolymers thereof and derivatives thereof. By derivative is understood such polymers or copolymers which have been functionalized by a suitable functional group, while maintaining the degradable and biocompatible properties. Preferred polyesters are aliphatic polyesters. It is an advantage of embodiments of the present invention that polyester fibres may enable a controlled speed of resorption through for example the choice of the polyester fibres molecular weight or through its composition in monomer units in case of polyester copolymers.

In embodiments, such a fibre blend also presents an increased hydrophilicity thanks to the presence of phosphoester units as compared to a pure polyester fibre. The increased hydrophilicity is for example observed by a decrease in contact angle when a drop of water is deposited on the fibre blend. The contact angle for a hydrophobic polyester fibre such as polycaprolactone on an aqueous surface is typically higher than 90° while the contact angle of the same polyester fibre containing phosphoester units may be lower than 20°, even lower than 10°. An increased hydrophilicity may be observed even when only a few phosphoester units are present in the copolymer fibre structure. An increased hydrophilicity is beneficial for applications as resorbable membranes in dental implants. It also helps increasing the softness in an aqueous environment. On the other hand, it is advantageous that the phosphoester units in the copolymer fibre of the fibre blend according to the invention are from 1 to 50 % in number versus the total number of monomer units in said copolymer fibre, so that the fibre blend remains insoluble in an aqueous environment, such as in water. Preferably, the amount of phosphoester units in the copolymer fibre is 40% or less, more preferably 30% or less, most preferably between 10 and 20%.

Furthermore, phosphoester units in the copolymer fibre of the fibre blend according to the invention are advantageous to favour bone regeneration. Phosphoester units may modulate the surface properties of the fibre. For example, surface properties may be such that undesired cells do not adhere on the fibres. As another example, surface properties may be such that it favours interaction with other molecules. Due to its pentavalency, the phosphorous atom in phosphoester units offers a physico-chemical versatility. It also enables different functionalities. For example, it enables the introduction of a negative charge on an oxygen atom linked to said phosphorous atom, thereby offering possibilities of electrostatic interactions with positive charges of other compounds. Furthermore, polymers or copolymers made from phosphoester units (PPE) are hydrolytically degradable. Additionally, PPE is often referred to as DNA-like, because phosphodiester is indeed a building block of DNA or RNA. Based on this chemical structure it has been consistently reported as biocompatible even *in vivo.*

Phosphoester units are preferably selected from the list consisting of alkyl-H-phosphonate, phosphoramidate, phosphonate, phosphate and the like. Phosphoester units may be obtained by the ring-opening of the corresponding cyclic phosphate derivatives or cyclic phosphonate derivatives. Examples of cyclic monomers include 2-methoxy-2-oxo-1,3,2-dioxaphospholane, 2-ethoxy-2-oxo-1,3,2-dioxaphospholane, 2-propoxy-2-oxo-1,3,2-dioxaphospholane, 2-butoxy-2-oxo-1,3,2-dioxaphospholane, 2-(but-3-en-1-yloxy)-1,3,2-dioxaphospholane 2-oxide, 2-(but-3-yn-1-yloxy)-1,3,2-dioxaphospholane 2-oxide, 2-(allyloxy)-1,3,2-dioxaphospholane 2-oxide, 2-(vinyloxy)-1,3,2-dioxaphospholane 2-oxide, such as for example described in Macromol. Biosci. 2016, 16, 1745-1761.

It is an advantage of embodiments of the present invention that the fibre blend according to the invention is porous. In embodiments, the fibre blend has a high surface/volume ratio and a high porous volume. In particular, the fibre blend presents a porosity with interconnected pores. Such properties enable an air or vapor permeability, an interesting property that may contribute to the healing of tissue. In the application of bone regeneration, such permeability may further allow nutriments for example to traverse the fibre blend or even colonize the fibre blend, and participate in the regeneration of bone tissue. On the other hand, the fibre blend will act as a barrier preventing undesired cells or bacteria to enter. The fibre blend may also prevent the migration of the soft tissues into the space to be regenerated so as to allow the more slowly growing bone tissue to colonize the volume created. The fibre blend according to the invention is particularly suitable for such barrier effect.

It is an advantage of embodiments of the present invention that the fibre blend is easy to sterilize. It may for example be sterilized by UV radiation, gamma radiation, beta radiation, immersion in ethanol, ethylene oxide, chlorite anion or by supercritical carbon dioxide. In embodiment, it is also advantageously cytocompatible.

In embodiments, the degradable polyester fibre in the fibre blend according to the first aspect of the invention may represent at least 50% by weight of the blend.

This is advantageous to maintain the high mechanical resistance provided by the polyester fibre. In preferred embodiments, the degradable polyester fibre represents at least 75% by weight of the blend, preferably at least 80% or even at least 90%.

In embodiments, the copolymer fibre represents at least 1% by weight of the blend, preferably at least 5 %, more preferably at least 10%.

In embodiments, the copolymer fibre in the fibre blend according to the first aspect of the invention may be a block copolymer comprising a block of degradable ester units and a block of phosphoester units.

This is advantageous to maintain the physico-chemical properties of each polymer in the copolymer, as for example the glass transition temperature and/or the crystallinity. It also favours the anchoring of the phosphoester units within the blend. It is also advantageous to facilitate the preparation of the copolymer fibre.

In preferred embodiments, the degradable polyester fibre in the fibre blend according to the first aspect of the invention is a polycaprolactone. This aliphatic polyester presents good mechanical properties as well as appropriate degradability properties so as to allow sufficient time for bone regeneration and then be resorbable. Preferably, the degradable ester units in the copolymer fibre of the fibre blend of the invention are the same as the ester units of the degradable polyester of the fibre blend. This is advantageously to confer a good miscibility between the polyester fibres and the copolymer fibres. Preferably said ester units are caprolactone units. Alternatively, the degradable polyester fibre may itself be a copolymer with two or more degradable ester units. It may for example be the copolymer polylactic-co-glycolic acid.

In embodiments, the phosphoester units in the copolymer fibre of the fibre blend according to the first aspect of the invention may be units according to one or more of formula (I), (II), or (III)

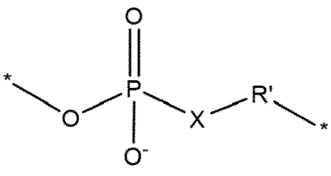

Formula (I)

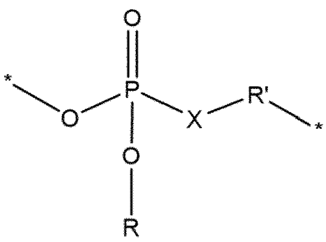

Formula (II)

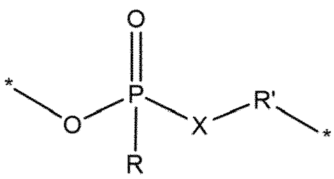

Formula (III)

wherein:

X represents an oxygen or a $-CH_2$ or a -NH group,
R represents a hydrogen, an alkyl group of from 1 to 10 carbon atoms, a substituted alkyl group of from 1 to 10 carbon atoms, an allyl group, or an alkoxy group of from 1 to 10 carbon atoms;
R' represents an alkylene group of from 1 to 10 carbon atoms or a substituted alkylene group of from 1 to 10 carbon atoms.

Preferably, at least some phosphoester units according to Formula (I) are present in the copolymer fibre, bringing negative charges at the surface of the fibre blend. Such negative charges may be balanced by lithium ions, sodium ions,

potassium ions or any suitable ions.

In preferred embodiments, phosphoester units are phosphate units. In more preferred embodiments, phosphoester units are obtained from allyl cyclophosphate, followed by the removal of the allyl function on the phosphate so as to release the negative charges on the phosphate units. This release of negative charges on the phosphate is also referred to as deprotection. In some case the deprotection may be partial. For example, the deprotection may be from 50 % to 100 %, preferably from 75 to 95 %. The degree of deprotection may be measured by $^1H$ NMR analysis. In some cases, deprotection may be performed during the preparation of the fibre blend, thereby not necessitating a particular step. Other phosphate monomers may be used, with other type of protection on the negative charge of the phosphate units.

In embodiments, the copolymer fibre in the fibre blend according to the invention is according to formula (IV)

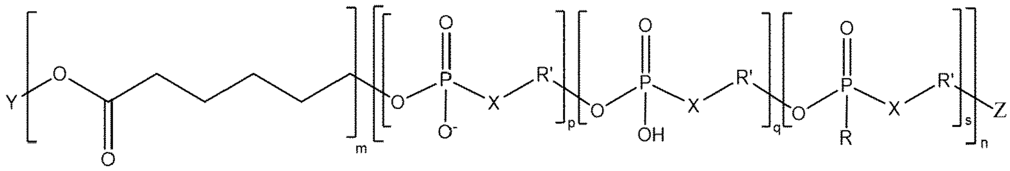

Formula (IV)

wherein a block of caprolactone units (left in formula IV) is attached to a block of phosphoester units (right in formula IV), such phosphoester units being randomly distributed in such block of phosphoester units;

X in formula (IV) represents an oxygen or a $-CH_2$ or a -NH group;
R in formula (IV) represents a hydrogen, an alkyl group of from 1 to 10 carbon atoms, a substituted alkyl group of from 1 to 10 carbon atoms, an allyl group, or an alkoxy group of from 1 to 10 carbon atoms;
R' in formula (IV) represents an alkylene group of from 1 to 10 carbon atoms or a substituted alkylene group of from 1 to 10 carbon atoms.
m in formula (IV) defines the number of caprolactone units and is an integer from 2 to 80, preferably from 30 to 40;
n in formula (IV) defines the number of phosphoester units and is an integer from 1 to 40, preferably from 5 to 10;
p in formula (IV) defines the number of anionic phosphoester units, with $0 \leq p \leq n$;
q in formula (IV) defines the number of phosphoester units bearing a hydroxyl group, with $0 \leq q \leq n$;
s in formula (IV) defines the number of phosphoester units bearing a hydrogen, an alkyl, allyl or alkoxy group with $0 \leq s \leq n$; wherein $1 \leq p+q+s \leq 40$.
Y and Z represent any suitable group at copolymer fibre chain ends.

In most preferred embodiments, the copolymer fibre in the fibre blend according to the invention is according to formula (V):

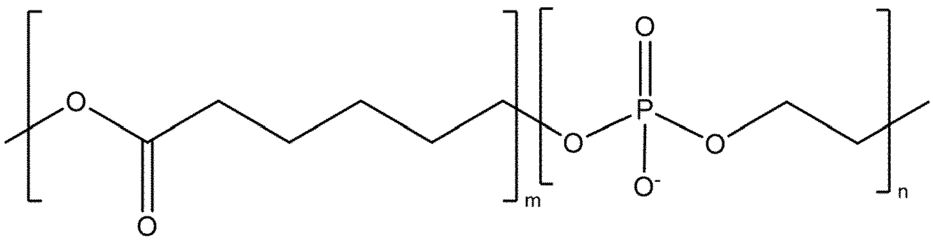

Formula (V)

wherein a block of caprolactone units (left in formula V) is attached to a block of ethyl phosphate units (right in formula V). m defines the number of caprolactone units and n defines the number of phosphate units. In preferred embodiments, m is an integer from 2 to 80, preferably from 30 to 40, and n is an integer from 1 to 40, preferably from 5 to 10. For example, the copolymer fibre may bear 35 units of caprolactone and 6 units of phosphate.

In embodiments, the diameter of the fibres in the fibre blend according to the first aspect of the invention may be from 0.01 to 100 micrometers, preferably from 0.1 to 10 micrometers. Such (sub)micrometric diameter allows for a very high surface/volume ratio. Advantageously, such diameter may be controlled by the process of preparation of the fibre blend, such as by controlling the voltage of electrospinning needle. The diameter of the fibres may be measured by electronic microscopy.

In embodiments, the fibre blend according to the first aspect of the invention may be coated by a polysaccharide.

The polysaccharide used to coat the fibre blend may for example be chitosan, hyaluronic acid, alginate or their

blends in any proportions.

In preferred embodiments, the polysaccharide is chitosan. This is advantageous due to the solubility of chitosan in water and its biocompatibility. Furthermore, chitosan may also confer antibacterial properties to the fibre blend. Preferably, the chitosan has a deacetylation degree above 50%, and a viscosity in 1% acetic acid of 0.8 - 2 Pa.s. It may have a molar mass of from 5000 g/mol to 850 000 g/mol.

Preferably, the fibres in the fibre blend may have a core-shell structure, with the polysaccharide being coated around the blend of degradable polyester fibres and degradable copolymer fibres.

In embodiments, the fibre blend according to the first aspect of the invention may comprise at least two layers of polysaccharide coating.

It is advantageous that the polysaccharide coating increases the rigidity of the fibre blend. In preferred embodiments, the fibres may have a bilayer structure, or a 3-layer structure, or a 4-layer structure or even more preferably a 5-layer structure. The number of layers may advantageously be adapted such as to obtain the desired mechanical properties. The rigidity is preferably such that the fibres may be easily cut in dry state while still being draping in wet state.

In preferred embodiments, the fibre blend according to the first aspect of the invention may further comprise a layer of poly(phosphoester) between two layers of polysaccharide. Such layers of polysaccharide and layer of poly(phosphoester) are coating the fibre blend.

This is advantageous to increase the adhesion of polysaccharide to the fibre blend. Advantageously, a poly(phosphoester) bearing negative charges may be in electrostatic interaction with positive charges of a polysaccharide such as chitosan. This avoids chemical modification of one or both polymers (eg. chitosan or polyester), thus preserving their properties. It is also advantageous that poly(phosphoester) is hydrophilic. Such a hydrophilic polymer favours an interaction with polysaccharide. In other embodiments, such as for example with hyaluronic acid as polysaccharide, an intermediate layer may be used between the polyester and the polysaccharide to favour polysaccharide coating on the polyester fibres.

A poly(phosphoester) may for example be a polyphosphate. It may for example contain from 50 to 500 monomer units or more.

It is an advantage of embodiments of the present invention that the thickness of the fibre blend may be increased while maintaining its degradability properties.

In embodiments, the fibre blend according according to the first aspect of the invention may further comprise calcium ions.

Calcium ions are known to favour bone regeneration. Advantageously, calcium ions may also improve the mechanical stability of the fibre blend. Advantageously, the phosphoester units may contain negative charges which may be lead to electrostatic interactions with positive charges of calcium ions. Thus calcium ions may be present as counter ions of phosphoester units. Alternatively, calcium ions may be present as a layer between the fibre blend according to the first aspect of the invention and a layer of poly(phosphoester) and/or between two layers of poly(phosphoester).

It is an advantage that a polysaccharide may be replaced by a layer of calcium ions.

According to a further aspect, the invention relates to a fibre blend according to the first aspect for use in a medical treatment. The fibre blend according to the invention has a permeability that allows nutriments to traverse and/or colonize it. When such fibre blend is used in contact with the tissue of a subject, the fibre blend may contribute in the healing of such tissue. The tissue of a subject may for example be a bone tissue. Healing of the tissue may for example be regeneration of bone tissue. The fibre blend has an effect of favoring such regeneration. Thus the fibre blend may be used in a method of treatment of bone tissue defects. In particular, the fibre blend may be used to treat periodontal defects, alveolar bone defects, sinus lifts.

According to a second aspect, the invention relates to a process for preparing a fibre blend which may comprise the steps of:

(i) providing a solution of a degradable polyester and a degradable copolymer, said copolymer comprising degradable ester units and from 1 to 50 % in number of phosphoester units versus the total number of monomer units in said copolymer,
(ii) electrospinning said solution so as to obtain a fibre blend,
(iii) optionally releasing negative charges of phosphoester units of the fibre blend,
(iv) optionally dipping the fibre blend in a polysaccharide solution so as to obtain a coated fibre blend,
(v) optionally dipping the coated fibre blend in a poly(phosphoester) solution and subsequently dipping the fibre blend in a polysaccharide solution so as to obtain a layered coated fibre blend,
(vi) optionally repeating step (v) until the desired layer number is reached.

It is advantageous to use electrospinning for the preparation of the fibre blend. Electrospinning is a liquid ionization technique to produce a solid fibre, in the nanometric or micrometric size. It is based on the uniaxial stretching or elongation of a viscoelastic jet derived from a polymer solution or melt. Unlike melt processing operations, this technique

enables the spinning of fibres from a polymer solution at room temperature, utilizing an evaporating solvent as the means to produce oriented fibres. Non-oriented fibres may also be obtained. A typical electrospinning setup comprises a high-voltage power supply, a needle spinneret and a conductive collector. Deposition of the fibres onto the collector forms a fibre mat.

Typical conditions for electrospinning are a voltage from 10 to 25 kV, a flow rate from 0.01 to 1.5 mL/h and needle with outer thickness of from 0.1 to 1 mm.

The duration of electrospinning may vary from a few minutes to several hours, for example 30 minutes to 6 hours. Advantageously, the duration enables to control the thickness of the mats formed by the fibre blend.

It is an advantage in embodiments of present invention that electrospinning produces fibres with a very small diameter (in the (sub)micrometric size), enabling a high surface/volume ratio and a large porosity with interconnected pores. Advantageously, the diameter can be controlled with the voltage of electrospinning device. For example, higher voltage may decrease the fibre diameter.

Electrospinning is typically performed from a solution of polymer. The polymer is therefore provided in a suitable solvent. Suitable solvents for polyesters may be organic solvents such as dimethyl formamide or dichloromethane or a mixture of both. Suitable solvents may also include acidic solutions such as acetic acid or formic acid or a mixture of both. It is of common knowledge to choose an appropriate solvent depending on the polyester type. A typical concentration of the polymer in solution may be from 10 w/v % (weight % of polymer versus total volume of solution) to 20 w/v %, preferably from 14 to 17 w/v %.

After the electrospinning step, fibres are commonly referred to as mats or fibre mats.

To enable an optional coating of the fibres, the mats may be dipped in a solution of the desired polymer for coating.

Alternatively, co-axial electrospinning may be used, in which a polymer solution is driven in a first jet and a second polymer solution is driven in a second jet. A typical set-up of co-axial electrospinning may comprise a high-voltage power supply, a conductive collector and two-fluid stainless steel coaxial spinneret, which comprises two concentrically designed needles, one being the inner nozzle and the second being the outer nozzle. In this alternative embodiment, step (i) is adapted for providing a first solution of a degradable polyester and a degradable copolymer, said copolymer comprising degradable ester units and from 1 to 50 % in number of phosphoester units versus the total number of monomer units in said copolymer, in an inner nozzle of a co-axial electrospinning device; and a second solution of polysaccharide in an outer nozzle of said co-axial electrospinning device. Step (ii) is adapted for electrospinning both first and second solutions so as to obtain a core-shell fibre blend.

The solution injected through the inner nozzle forms the core of the fibre and the solution injected through the outer nozzle forms the coating or shell of the fibre. Core-shell fibres may hence advantageously be obtained in a one step process.

In one possible embodiment, the phosphoester units of the degradable copolymer provided in a solution according to step (i) of the process according to the second aspect of the invention are in a protected configuration before the step (ii) of electrospinning. In such a protected configuration, any negative charges of phosphoester units are protected by a so-called protecting group. By protecting group is meant any group playing the role of protection of a phosphate or phosphonate group during electrospinning and being able to be released in a deprotection step, thereby leaving a negative charge on the oxygen to which it is linked. The protecting group is for example a methyl, an ethyl, a benzyl, an allyl or a phosphoramidate group, preferably the protecting group is an allyl group.

Preferably, the phosphoester units in protected configuration are allyl phosphoester units.

When such a protecting group is used, step (iii) may be needed to release the negative charges of phosphoester units. Step (iii) is preferably done after step (ii), although step (iii) may also be concomitant with step (ii). In other embodiments, no negative charges are to be released. In yet other embodiments, a partial release may be done. In some embodiments, a partial release occurs naturally during the electrospinning step (ii). Hence, optionally releasing negative charges of phosphoester units of the fibre blend means a step enabling the (partial) removal of a protecting group on such phosphoester units.

The (partial) release of negative charges of phosphoester units is advantageous when a subsequent coating of the fibre blend by a polysaccharide bearing positive charges is desired. Negative charges may then be used to form an electrostatic interaction with a polysaccharide bearing positive charges. For example, chitosan is positively charged in an acidic medium.

The release of negative charges of phosphoester units of the fibre blend, also referred to as deprotection, may be performed by adding an agent capable of at least partially removing a protecting group on phosphoester units of the fibre blend. Such an agent is preferably a nucleophile agent, such as for example sodium iodide or sodium benzene thiolate. Preferably, the nucleophile agent is used in concentration from 1 to 3 equivalent versus the phosphoester functions. Preferably, the deprotection is performed in an aqueous solution.

In an optional step, the fibre blend is dipped in a solution containing a polysaccharide so as to obtain a polysaccharide-coated fibre blend. Such dipping is advantageously performed in an aqueous solution. Advantageously, the solution is an acidic solution. Preferably, the solution is an acidic chitosan solution. For example, the solution may be at

pH from 1 to 7, preferably at a pH from 1 to 6.

Before dipping, the fibre blend may previously be immersed in a slightly basic solution, such as at pH 8. The pH may for example be controlled by a suitable buffer, such as Tris buffer. Dipping the fibre blend in a polysaccharide solution so as to obtain a coated fibre blend may be with a duration of from 1 minute to several hours, preferably from 5 minutes to 1 hour, for example 30 minutes. Optionally, the coated fibre blend may subsequently be rinsed in water.

In a yet further optional step, the polysaccharide-coated fibre blend is successively dipped in a solution of a poly(phosphoester) and in a solution of polysaccharide in order to form a multi-layered fibre blend. When chitosan is used as a polysaccharide and an anionic poly(phosphoester) is used, there is an electrostatic interaction between the positively charged chitosan and the negatively charged poly(phosphoester). Alternatively, a buffer solution such as for example a buffer solution at pH 5 may be used when the polysaccharide is a hyaluronic acid solution, so as to compensate the weak acidity of the solution compared to the other polyanionic solutions.

Any feature of the second aspect may be as correspondingly described in any other aspect of the invention.

According to a third aspect, the invention relates to a membrane for bone regeneration comprising the fibre blend according to the first aspect.

In preferred embodiment, the membrane for bone regeneration may have a thickness of from 1 micrometer to 1 millimeter, preferably from 50 micrometers to 500 micrometers, more preferably from 100 micrometers to 300 micrometers.

Advantageously, said thickness of the membrane may be controlled by the duration of electrospinning. The longer the duration of electrospinning, the thicker the membrane may be. For example, the duration may be from 5 minutes to 24 hours, preferably from 30 minutes to 6 hours, more preferably from 1 hour to 3 hours. This is advantageous for the handling of the membrane, as evaluated qualitatively by clinicians.

Preferably, the membrane according to the invention is structured. This may be obtained by adapting the conductive collector of the electrospinning device with a structured collector. The obtained structure in the fibre blend may correspond to the structure of the collector. Different structures may be chosen, such as a grid or a honeycomb collector. This is advantageous to modulate the density of the membrane or fibre blend. For example, a higher density of fibres may be obtained on the edges of the structured collector and a lower density in the holes of the structured collector. This variable density of fibres may also create a gradient in the porosity of membrane or of the fibre blend.

This variable density/porosity has several advantages. First, when used in dental implants, it may allow for a better adherence between the membrane and the muco-periosteal flap, thereby limiting the occurrence of dehiscence. Secondly, it may play a role in the speed of resorption of the membrane. Advantageously, the membrane allows for a barrier functionality of at least 12 weeks before start of degradation. Thirdly, the variable fibre density is advantageous to prevent the passage of cells, referred to as cellular occlusion, while allowing the passage of molecules such as nutrients, metabolic products and the like. Furthermore, the membrane is efficient for the development of bone tissue located under this membrane.

The variation of density may be present along the longitudinal plane of the membrane and/or may be present along the thickness of the membrane. In embodiments, a lower fibre density on one face of the membrane may be advantageous as a support for the reconstruction of bone while a higher density on the second face of the membrane may provide more effective barrier properties to the migration of unwanted cells. Therefore, obtaining a membrane whose two faces are of different porosity, or present a gradient of density from one face to the other, is particularly advantageous.

More preferably the structure may be a honeycomb pattern. This is advantageous to bring good mechanical properties to the membrane. A higher density of fibres in the form of a honeycomb will confer an excellent mechanical strength.

A honeycomb pattern with different size of hexagonal hole may be used. For example, the hole diameter may be from 0.05 to 5 mm, preferably from 0.1 to 2 mm. A smaller hexagon size may lead to an increased stiffness of the membrane. A higher hexagon size may increase the wettability of the membrane.

In embodiments, the membrane may have two different faces. The two faces may vary in fibre density, as described here above, and/or the two faces may vary in chemical composition. For example, the membrane may have one face wherein the fibres have been coated with a polysaccharide and one face wherein the fibres have not been coated and/or which contains pure polyester fibres only. This may further modulate the properties as a function of the desired application. Thanks to the phosphoester units which can be located at desired places in the copolymer fibre, it may also be possible to selectively coat a polysaccharide on specific places of the membrane where phosphoester units are present.

Any feature of the third aspect may be as correspondingly described in any other aspect of the invention.

According to a fourth aspect, the invention relates to the membrane according to the third aspect for use in a medical treatment.

For example, the membranes may be used for bone regeneration of a human or animal subject, preferably bone regeneration refers to intraoral bone regeneration. For example, it may be used to treat periodontal defects, alveolar bone defects, sinus lifts.

Any feature of the fourth aspect may be as correspondingly described in any other aspect of the invention.

## Examples

It is to be understood that although preferred embodiments, specific compositions and methods have been discussed herein for products according to the present invention, various changes or modifications in form and detail may be made without departing from the scope of this invention. For example, any formulas given above are merely representative of materials that may be used. Steps may be added or deleted to methods described within the scope of the present invention.

### Example 1. Preparation of polycaprolactone (PCL) fibres

PCL fibres were prepared by electrospinning in a mixture of dimethylformamide (DMF) and dichloromethane (DCM) as solvent. An uniform fibre size was obtained with the parameters summarized in Table 1, namely a polymer concentration of 14 or 17 % (weight/volume), a voltage (V) between 10 and 20 kV, a flow rate (F) between 0.5 and 1.5 mL/h, a collector having a flat plate or a honeycomb structured plate, an emitter having a 0.4 to 0.8 mm needle diameter. The needle tip to collector distance was between 15 and 20 cm and the temperature was 25 °C.

Table 1: Electrospinning conditions and PCL fibres diameter

| Sample | Conc. (%) | V (kV) | F (mL/h) | Collector | Diameter (μm) | |
|---|---|---|---|---|---|---|
| | | | | | Internal | Wall |
| A | 17 | 13 | 1 | Honeycomb | 0.563 | 1.219 |
| B | 17 | 18 | 1 | Honeycomb | 0.605 | 0.883 |
| C | 17 | 13 | 0.6 | Honeycomb | 0.476 | 1.132 |
| D | 14 | 13 | 1 | Honeycomb | 0.547 | 1.064 |
| E | 17 | 13 | 1 | Flat | / | 1.114 |
| F | 17 | 18 | 1 | Flat | / | 0.688 |

From Table 1, it can be observed that a fibre diameter between 0.4 and 1.3 μm was obtained. The wall diameter is decreased when a higher voltage is used and/or when a honeycomb collector is used. A lower flow rate also decreases the fibre diameter although to a lesser extent.

Samples E and F were used for the further characterisation of the fibres. Results of physico-chemical tests are presented in Table 2 and the techniques are detailed hereafter.

Table 2: characteristics of PCL fibres

| Parameter | Technique | Sample E | Sample F |
|---|---|---|---|
| Thickness | Micrometer | 100 ± 10 μm | 80 ± 10 μm |
| Porous volume | Impregnation of a liquid | 92 % | 92 % |
| Pore size | Capillary flow porosimetry | 4.0 ± 0.1 μm | 2.7 ± 0.1 μm |
| Air permeability | ISO 9237 | 52 $L/m^2.s$ | / |
| Vapor permeability | ISO 11092 | 4.6 $m^2$. Pa/W | / |
| Bacteria barrier property | ISO 22610 | 3.7 | / |

For the measurement of through-pore size by capillary flow porometry, air was passed through a dry sample and then through the same sample wetted with a liquid of known surface tension. The air flow rate through the membrane is measured as a function of pressure for both samples, allowing the through-pore size to be determined. For air permeability, the technique consists in applying a certain pressure between the two sides of the membrane and to measure the air flow rate going through. The membrane resisted to the test. For vapor permeability (also called Thermal Evaporative Resistance), the sample is placed on a heated porous plate. Water is fed to the heated plate and evaporates. The vapor passes through the membrane as vapor. The heat flux required to maintain a constant temperature at the plate is a measure of the rate of water evaporation. From this the water-vapor resistance of the sample is determined. The membrane resisted to the test. The sample can be classified as extremely breathable ($R_{et}$ < 6 $m^2$. Pa/W). For barrier properties to bacteria, the sample is placed on an agar plate. A sheet carrying bacteria is then placed on top of it with the

contaminated side facing down. A metallic finger is placed on top of the materials with a specified force to bring the test specimen into contact with the agar surface. The finger is moved over the entire surface. Due to the combined effect of rubbing and liquid migration up from the agar surface, bacteria may pass from the donor material through the sample down to the agar surface. The agar plates are incubated to grow the bacterial colonies, which are then counted. The membrane resisted to the test. The barrier index, $I_B$, was 3.7. According to the requirements of EN13795 (2019) for medical textiles, the sample fully complies with the standard performance of use ($I_B$ > 2.8).

**Example 2: preparation of polycaprolactone-block-poly(allyl phosphate) copolymer (PCL-*b*-PPE)**

Firstly, a hydroxyl PCL macroinitiator was synthesized (PCL-OH). Therefore, before polymerization, caprolactone (monomer) and benzylic alcohol (initiator) were both dried on calcium hydride, distilled under vacuum and stored under inert atmosphere. 14 mL (123 mmol) of caprolactone is transferred into a flamed and nitrogen-purged glass flask via a freshly flamed and nitrogen purged stainless steel capillary. 0.4 mL (3.7 mmol) of benzylic alcohol is transferred into the flask via a freshly flamed and nitrogen purged stainless steel capillary. The glass flask is then put in an oil bath at 110°C and 7.2 mL of a tin octoate solution in toluene ([C] = 0.06 mol/L) is added via a freshly flamed and nitrogen purged stainless steel capillary. The solution is stirred for 100 minutes at 110°C. The PCL-OH macroinitiator is recovered by solubilization in dichloromethane followed by a precipitation in cold heptane. After filtration, a white powder is collected.

Secondly, PCL-*b*-poly(allyl phosphate) copolymer was synthesized. Therefore, 4 g (1 mmol OH) of hydroxyl PCL macroinitiator and 0.188 g (0.5 mmol) of *N*'-[3,5-Bis(trifluoromethyl)phenyl]-*N*-cyclohexylthiourea (thiourea) were poured into a flamed and nitrogen-purged glass flask via a freshly flamed and nitrogen purged stainless steel capillary and dried by three successive azeotropic distillations with anhydrous toluene. 1 g (6 mmol) of freshly distilled allyl cyclophosphate monomer is transferred into the flask and put under vacuum during 15 minutes before the addition of 40 mL of anhydrous dichloromethane via a freshly flamed and nitrogen purged stainless steel capillary. The glass flask is then put in an ice bath at 0°C and 0.2 mL (1.3 mmol) of 1,8-diazabicyclo[5.4.0]undec-7-ene is added via a freshly flamed and nitrogen purged stainless steel capillary. The solution is stirred for 60 min at 0°C. The PCL-*b*-poly(allyl phosphate) copolymer is precipitated in cold diethyl ether, recovered by filtration and dried under vacuum.

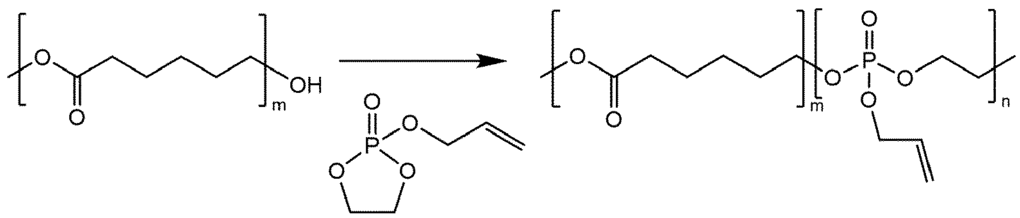

Scheme 1: synthesis of block copolymer PCL-*b*-PPE

The successful synthesis of copolymer PCL-*b*-PPE was shown by $^1$H NMR and size exclusion chromatography. The number of caprolactone units was evaluated to be 35 units and the number of phosphate units was 6 units. The phosphate groups in PCL-*b*-PPE diblock copolymer were then deprotected. The deprotection step may be done during electrospinning, previously to electrospinning or after electrospinning. When done in an organic solvent before electrospinning, the copolymer containing the allyl protecting group was first stirred with 3 eq. of nucleophile agent (i.e., sodium iodide or sodium benzene thiolate) in DMF at room temperature for 6 hours. Then, the reaction mixture was concentrated under vacuum and purified by precipitation in cold diethyl ether to remove the allyl-nucleophile by-product (i.e., allyl-I or allyl-SPh).

**Example 3: Preparation of fibre blend PCL/PCL-b-PPE**

Several electrospun PCL mats containing PCL-*b*-PPE diblock copolymer were prepared. Electrospinning conditions were first optimized and kept constant for each polymeric system. These conditions were solvent (DCM/DMF 50/50 (v/v)), polymer (PCL/PCL-b-PPE 9:1 (w/w)), concentration (15 % (w/v)), flow rate (10-13 μL/min), collector (flat aluminum plate, honeycomb, tubular drum), needle (27 G), needle tip to collector distance (15-20 cm), temperature (25 °C), and voltage (15-25 kV).

Fibres were characterized by using contact angle measurements. Indeed, the presence of phosphate groups may increase hydrophilicity. For this, the PCL/PCL-*b*-PPE fibres were primarily immersed in pH = 8 PBS buffer for 30 min and dried under reduced pressure at ambient temperature for 2 days prior to analysis. Contact angles were measured by the Sessile Drop technique at 20°C using a GBX Digidrop instrument (DGD Fast 60 Contact Angle Meter). A 3 μl distilled water droplet was dropped onto the fibre mat surface and temporal images of the droplet were taken via a camera, 20 s

after droplet deposition. The contact angle was then calculated by WinDrop software on the obtained images. The measurement was repeated 5 times on each fibre mat sample. The results are shown in Figure 1, with contact angle measurement on PCL fibre (a) and on PCL/PCL-*b*-PPE fibre (b). The contact angle measured on pure PCL fibre sample after immersion in pH = 8 in the same condition is much higher (i.e., 115°, Figure 1a). In contrast, in the presence of PCL-*b*-PPE copolymer in the fibre blend, the measured contact angle of water is very low (i.e., about 8°, Figure 1b). In principle, the contact angle of a water droplet on a surface reflects the hydrophilic character of the surface despite possible influence of the surface roughness. The pronounced variation from hydrophobicity to hydrophilicity confirms the presence of the PCL-*b*-PPE copolymer (i.e., the presence of the phosphate groups) on the surface of fibres.

Fibres were also characterized in terms of stability and mechanical properties over time. Dynamic mechanical analyses (DMA) were performed on a stress-controlled DMA instrument (Q800, TA Instruments), under the tensile/extension mode. For the analyses, an engineering creep ramp (by a stress sweep from 0.0 - 3.0 *MP* of static stress) with a ramp rate of $\delta\sigma/\delta t = 0.0100\ MPa/min$ were performed on each rectangular specimen with about 5 mm of width, length of about 10 mm and varied thickness of 60 μm to 150 μm. The measurement was done at ambient temperature to generate the classical stress-strain curve. The evolution of the Young modulus during ageing for 6 months in a phosphate buffer at 37°C, under agitation, was monitored with three samples prepared by conditions A and B (Table 1, with honeycomb structure). Two types of membranes were tested with different wall fibre diameter (A: 1.11 μm and B: 0.69 μm) and three samples of each were evaluated for reproducibility. Results are presented in Figure 2, 3 and 4.

The mechanical properties of the fibres during the first 6 months were stable, an elastic modulus varying between 6 and 8 MPa was obtained, with a decrease during the last two months for sample B, which had a smaller wall diameter. The elongation at break remained around 200% apart from an increase in the latter over the last two months for sample B. This may be due to hydrolytic degradation of the PCL chains which decreases the physical crosslinking nodes by reducing the molar mass chains.

There was no significant difference within the electrospun layer in any evaluation time. This feature is probably due to the higher crystallinity degree of such PCL used in this study (50.3%, calculated by DSC curves). This structural integrity for 6 months is an excellent indication of the potential use of a fibre blend according to embodiments of the invention in a medical treatment, such as bone regeneration in which the fibre blend should remain functional until the successful healing process of the bone defect.

**Example 4: synthesis of poly(phosphoester) homopolymer and its deprotection ($PPO^-$)**

The synthesis of the poly(phosphoester) homopolymer ($PPO^-$), negatively charged polyelectrolyte, was targeted following a step reported by Clement et al. in Macromolecule 2012, 45, 4476-4486. Typically, 48 mg of tetraethylene glycol (0.5 mmol of OH group) and 0.33 g (0.9 mmol) of N'-[3,5-Bis(trifluoromethyl)phenyl]-N-cyclohexylthiourea were poured into a flamed and nitrogen-purged glass flask and dried by three successive azeotropic distillations with anhydrous toluene. 4 g (24 mmol) of freshly distilled allyl cyclophosphate monomer was transferred into the flask and put under vacuum during 15 minutes before the addition of 20 mL of anhydrous dichloromethane via a freshly flamed and nitrogen purged stainless steel capillary. The glass flask was then put in an ice bath at 0°C and 0.4 mL (2.6 mmol) of 1 ,8-diazabicyclo[5.4.0]undec-7-ene solution was added via a freshly flamed and nitrogen purged stainless steel capillary. The solution was stirred for 30 min at 0°C. The poly(allyl phosphate) was precipitated in cold diethyl ether, recovered by decantation and dried under vacuum.

A homopolymer with allyl functions with a degree of polymerization of 96 was obtained and characterized by $^1H$ NMR, $^{31}P$ NMR and size exclusion chromatography.

The deprotection of the poly(allyl phosphate) was carried out as follows: the poly(allyl phosphate) was stirred with 1.5 eq. of nucleophile agent (i.e., sodium iodide or sodium benzene thiolate) in 20 mL of DMF at room temperature for 3 h. Then, the reaction mixture was concentrated under vacuum and purified by precipitation in cold diethyl ether to remove the allyl-Nu by-product (i.e., allyl-I or allyl- SPh). The sample was dissolved in DMF and dialyzed against Milli-Q water using Spectra/Por® dialysis membrane with 1000 MW cut-off. The sample was recovered by freeze drying and characterized by $^1H$ and $^1H$ decoupled $^{31}P$ NMR in $D_2O$.

**Example 5: coating of the fibres by a polysaccharide (CS) using the deposition method**

Chitosan (CS) was chosen as a polysaccharide. Coating of chitosan on the negatively charged PCL/PCL-*b*-PPE fibres was performed by electrostatic deposition. For that purpose, PCL-*b*-PPE diblock copolymer was first deprotected using NaI in an aqueous solution. Then, the charged fibres were immersed for 1 min in a phosphate buffer solution (pH=8) to obtain negatively charged phosphate groups. Afterwards, fibres were dipped in a chitosan solution (5 g/L in 0.1 M NaCl aqueous solution containing 1 % (v/v) of glacial acetic acid) for 30 min and rinsed in water for 1 min.

Figure 5 shows representative SEM images of fibres after chitosan deposition (left) compared to before chitosan deposition (right). It can be observed that the morphology of the coated fibres remains unchanged compared to the fibres before chitosan deposition. The mechanical properties are shown in Table 3.

Table 3: Mechanical properties of the different membranes

| Sample | Young modulus E (Mpa) | Strain σ (MPa) | Elongation at break ε (%) |
|---|---|---|---|
| PCL-A436 | 7.96 | 1.72 | 212 |
| PCL/PCL-b-PPE | 4.49 | 2.13 | 115 |
| PCL/PCL-*b*-PPE/CS | 80.00 | » 3.00 | << 8.00 |
| Commercial membrane | 18.6 | 2.75 | 30 |

The presence of the 10 weight% of PCL-*b*-PPE in the PCL fibres has a limited effect on the mechanical properties of the membrane as compared to pure PCL membrane. The observed mechanical properties are close to the value measured with a commercially available membrane. After the deposition of one chitosan layer, a significant increase of the stiffness of membrane at the dry state is obtained which facilitates the membrane handling and its cutting.

**Example 6: preparation of multilayered fibres**

The preparation of fibres comprising a core of PCL/PCL-*b*-PPE and a shell of multilayered chitosan was carried out. In this experiment, a fluorescent chitosan probe (FC) was used. The labelling of chitosan was obtained by reacting the primary amine group of the D-glucosamine on chitosan backbone with isothiocyanate group of fluorescein isothiocyanate in DMSO solvent for 15 hours in the dark at room temperature. This labelling is a method for fluorescent tracking of a chitosan-coated electrospun membrane. The basis of this tracking system is a fluorescence emitting with a quantum yield comparable to the original fluorescent dye. With fluorescence spectroscopy and microscopy being extremely sensitive analytical techniques, the addition of such fluorescent probe has been used for quantitative monitoring of an average intensity for multilayer coatings and nanostructured chitosan thin films.

Aqueous solutions of polymer were first prepared. Fluorescent chitosan was dissolved at a concentration of 5 g/L in 0.1 M NaCl aqueous solution containing 1 % (v/v) of glacial acetic acid.

Then, the layer-by-layer deposition of chitosan and poly(phosphoester) (PPO) on the surface of the PCL/PCL-*b*-PPE negatively charged fibre blend was conducted as follows: fibre mats were dipped in the chitosan solution for 30 min, rinsed in water for 1 min to form the chitosan mono-layered system. From this step, bilayers were deposited by alternatively dipping the fibre mats in the polyanionic solution for 30 min, rinsing them for 1 min, dipping them in chitosan solution for 30 min, and rinsing them for 1 min. The latter step was repeated 3 times to form the chitosan quintuple-layered fibre blend. The samples were then dried under reduced pressure at ambient temperature for 2 days.

The thickness of the membranes was measured by profilometer and values obtained are summarized in Table 4. SEM images are shown in Figure 6 (5-layers fibre blend PCL/PCL-*b*-PPE (left) and same fibre blend before chitosan coating (right). No significant difference was observed.

Table 4: Characteristics of PCL/PCL-b-PPE fibres

| Sample | mass (mg) | thickness (mm) |
|---|---|---|
| PCL/PCL-*b*-PPE | 50.2 | 0.28 |
| with 1 layer CS | 52.4 | 0.27 |
| with 5 layers CS | 56.0 | 0.32 |

Fibres comprising a core of PCL/PCL-*b*-PPE and a shell of multilayered chitosan were further characterized by fluorescence microscopy. Images were captured using an inverted Olympus IX81 microscope equipped with a Photometrics CoolSnap HQ CCD camera (C-BUN-F-XC50). Fluorescence images were illuminated with an Hg lamp (EXFO, series 120 PC Q) and a specific filter. The fluorescence emission increased dramatically once the density of FC increased on the surface of the fibres. It was also qualitatively observed that the quintuple-layered FC exhibited a strong fluorescence emission at 480 nm compared to its reference counterpart at this specific excitation. Figure 7 shows a fluorescence microscopy image of the section of fibre blend PCL/PCL-*b*-PPE after deposition of 5 FC/$PPO^-$ bilayers (appearing lighter on the image).

The fluorescence mean intensity (FMI) increased dramatically once the FC layer was deposited (Table 5).

Nevertheless, a small increase of the fluorescence was observed for the PCL-*b*-PPE fibres compared to the neat PCL. It is important to note that increasing the FC layer could improve the surface adhesion between PCL fibre and FC layer. In another words, the surface improvement of PCL is a main reason for increasing FMI through the layer-by-layer deposition of FC and $PPO^-$ on the surface of the charged fibres.

Table 5: Fluorescence mean intensity (FMI) for each membrane in the fluorescent zone

| Sample | FMI (mW $cm^{-2}$) |
|---|---|
| PCL | 4.54 |
| PCL/PCL-*b*-PPE | 22.06 |
| PCL/PCL-*b*-PPE + FC | 95.26 |
| PCL/PCL-*b*-PPE + 5 (FC/$PPO^-$) | 52.04 |

**Zeta potential measurements**

The surface of the fibres was characterized by using Indirect Zeta potential measurements on fibre surfaces, using the flat surface cell of a Beckman Coulter Delsa Nano C analyzer. The data were processed using the Delsa Nano UI 2.21 software. All the measurements were carried out at 25°C at a measurement angle of 15°, with standard particles for solid samples (Otsuka Electronics co., LTD, A54496). For the analysis, fibre mats electrospun for 3 hours were peeled off the aluminium foil and cut with scissors to fit into the analyzing flat surface cell. Measurement of the Zeta potential was repeated on five different samples from the same batch to check the reproducibility of the obtained results.

Figure 8 shows the Zeta potential results of the different fibres including PCL, PCL/PCL-*b*-PPE, PCL/PCL-*b*-PPE-, PCL/PCL-*b*-PPE/CS, PCL/PCL-*b*-PPE/CS/$PPO^-$, and PCL/PCL-*b*-PPE/CS/PPO-/CS. The reference fibres made of pure PCL-which is neutral- exhibit a negative Zeta potential close to zero. The observed slightly negative Zeta potential is explained by the presence of a few carboxylic acid functions formed along the fibres due to partial hydrolytic degradation of the aliphatic polyester. Accordingly, the PCL fibres containing the PCL-*b*-PPE copolymers possessed a negative Zeta potential. The presence of the PPE block caused the significant change of electrical charges throughout the PCL fibres. Remarkably, when the block copolymers were deprotected, the negative amount increased, highlighting the excess number of negative ions throughout the fibres. On the contrary, depositing a layer of the polycationic chitosan on the surface of negatively charged fibres changed the sign of the Zeta potential of the electrospun fibre mats. Interestingly, once this mat was dipped in polyanionic solution such as poly(phosphoester), the amount of Zeta potential decreased which is evidence of decreasing of the positively charged of the surface due to the presence of negatively charged poly(phosphoester) layer. Remarkably, the Zeta potential for the last sample which contained the second layer of chitosan had significant changes toward the positive values which is the evidence of good electrostatic interactions between the two polycationic and polyanionic layers also in comparison with the single layer of chitosan deposition.

**XPS measurement**

XPS analysis was performed to confirm the presence of the PPE at the surface of the electrospun blend after electrospinning and to evidence if the deprotection step keeps PPE at the surface of the nanofibers.

XPS spectra of an electrospun membrane of pure PCL (PCL) were compared to a membrane containing 10 weight% of PCL-*b*-PPE copolymer detached from the collector (PCL/PCL-PPE) and after deprotection by immersion in sodium iodide (PCL/PCL-PPE after dep.)

The chemical elemental surface composition of the electrospun fibre mats was determined by an SSI 100/206 photoelectron spectrometer from Surface Science Instruments equipped with a monochromatized microfocused Al X-ray source (powered at 20 mA and 10 kV). The pressure in the chamber was around $10^{-6}$ Pa. To perform XPS analysis, circular samples were fixed with double-sided tape onto small brass bores of 6 mm diameter that were placed on a ceramic carousel.

XPS graphs without chitosan coating are shown in Figures 9 and 10. The presence of the PPE based-copolymer (P peak) for both blend samples before and after deprotection is present, while it is absent on the pure PCL sample. These XPS data evidenced that the PCL-*b*-PPE block copolymer addition to the PCL nanofibres leads to the presence of phosphate groups detected at the surface of the fibres.

XPS graphs after chitosan coating are shown in Figures 11 and 12. If some chitosan is deposited on pure PCL fibres (figure 12B upper graph), quantification shows that the amount is significantly lower than on the two other samples containing the copolymer with phosphoester units (figure 12B middle and lower graph). Looking at the N1s deconvolution, we can conclude that 50% of the amine of the chitosan are protonated and that the interactions with the PPE containing

fibres is significantly different from pure coated PCL for which all amine are unprotonated. These data also evidenced that both PPE containing membranes are quite similar, the deprotection step having a low impact on the results. In these samples, the P/C ratio gives respectively a content of 18.9 and 14.9% of PPE which is quite close. The deposition of chitosan on the electrospun blend was successful and the deprotection step appeared to have a limited influence on the resulting composition.

**Example 7: preparation of membranes with modulated porosity and qualitative evaluation**

A collector structured in honeycomb was used for the electrospinning of the fibres. With such collector, a high density of fibres is obtained on the edges of the honeycomb and a lower density is obtained in the alveoli, as can be seen on the SEM images of sample A presented in Figure 13.

Several membranes were also evaluated qualitatively by the manipulation by clinicians. Membranes were based on fibre blend PCL/PCL-PPE with 3 types of surface patterns: smooth (with a flat collector), small honeycomb (hole diameter 0.5 mm), and large honeycomb (hole diameter 5 mm). The first criterion was their "rigidity" when dry, to facilitate the use of scissors. All three membranes behaved as commercial membranes. The second criterion was their hydrophilicity since barrier membranes have to be wet when placed into the patient. Here, the membranes with large honeycombs were more easily wettable. The third criterion was their draping effect since the barrier membrane should perfectly adhere to the bone tissues. The membranes with large honeycombs were associated with a better draping effect. Finally, the stretchability was evaluated, to test if the barrier membranes are elastic and resist stretching. Therefore, wet membranes were stretched by hand. All three types of membranes behaved similarly and succeeded in stretchability tests.

**Example 8: preparation of polyester-polysaccharide fibres by coaxial electrospinning**

In an alternative example, PCL/PCL-PPE$^-$/CS-PEO were prepared in a co-axial electrospinning setup. PCL/PCL-PPE$^-$ and CS-PEO form the core and the shell layer respectively. The set-up was as follows: a two-fluid stainless steel coaxial spinneret were used consisting of two concentrically designed needles. The inner and outer diameters of the inner needle were 1.07 and 1.47 mm, respectively, and the inner and outer diameters of the outer needle were 0.508 mm and 0.813 mm, respectively. The flow rates in the syringes were controlled by two separate pumps. The coaxial spinneret was connected to a DC high voltage power supply as a positive charge.

**Example 9: evaluation of the occlusive properties of the membrane**

The objective of this example was to evaluate barrier properties of the membrane, i.e. its capacity to isolate a bone regeneration chamber from the soft tissue while still allowing nutrients to participate in the bone regeneration treatment. The setup of this *in vitro* test was composed of Cell Crowns (Sigma-Aldrich), a device enabling the maintenance of membranes at a liquid interface, placed onto cell plates. Membranes were fixed between the insert and the crown. Murine fibroblasts (L-929 cell line) were stained with CFSE (carboxyfluorescein succinimidyl ester). For each well, $4\times10^5$ cells were incubated for 24h onto the membranes. The passage of fluorescent cells was controlled by confocal microscopy. Three types of PCL/PCL-*b*-PPE membranes structured in honeycombs with 3 different times of electrospinning (1, 3, and 5 hours) were tested. The higher the time of electrospinning the better occlusive the membrane may be. The positive control (allowing the passage of cells) was a commercial membrane with similar properties as Boyden membranes. The negative controls consisted of a commercial membrane largely used in clinics (BioGide, Geistlich, that is composed of porcine collagen. The positive control allowed the passage of some cells while all other membranes displayed no cells at the lower interface of the membranes, suggesting that the occlusive property was achieved in all membranes prepared according to the invention.

## Claims

1. A fibre blend for bone regeneration comprising at least a degradable polyester fibre and a degradable copolymer fibre, said copolymer fibre comprising degradable ester units and from 1 to 50 % in number of phosphoester units versus the total number of monomer units in said copolymer fibre.

2. The fibre blend according to claim 1 wherein the degradable polyester fibre represents at least 50% by weight of the blend.

3. The fibre blend according to any one of the preceding claims wherein the copolymer fibre is a block copolymer comprising a block of degradable ester units and a block of phosphoester units.

**4.** The fibre blend according to any one of the preceding claims wherein the degradable polyester fibre is a polycaprolactone and the degradable ester units are caprolactone units.

**5.** The fibre blend according to any one of the preceding claims wherein the phosphoester units are units according to one or more of formula (I), (II), or (III)

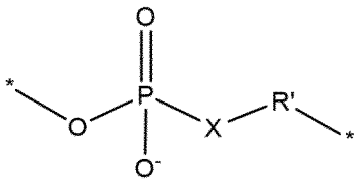
Formula (I)

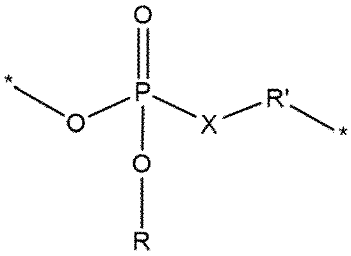
Formula (II)

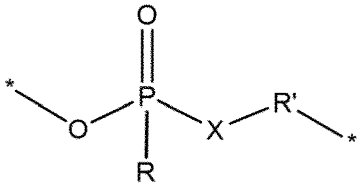
Formula (III)

wherein:

X represents an oxygen or a $-CH_2$ or a -NH group,
R represents a hydrogen, an alkyl group of from 1 to 10 carbon atoms, a substituted alkyl group of from 1 to 10 carbon atoms, an allyl group, or an alkoxy group of from 1 to 10 carbon atoms;
R' represents an alkylene group of from 1 to 10 carbon atoms or a substituted alkylene group of from 1 to 10 carbon atoms.

**6.** The fibre blend according to any one of the preceding claims wherein the diameter of the degradable polyester fibres and of the copolymer fibres is from 0.01 to 100 micrometers, preferably from 0.1 to 10 micrometers.

**7.** The fibre blend according to any one of the preceding claims being coated by a polysaccharide.

**8.** The fibre blend according to the previous claim wherein the polysaccharide is chitosan.

**9.** The fibre blend according to any of claim 7 or 8 comprising at least two layers of polysaccharide coating.

**10.** The fibre blend according to the previous claim further comprising a layer of poly(phosphoester) between two layers of polysaccharide.

**11.** The fibre blend according to any one of the preceding claims further comprising calcium ions.

**12.** A process for preparing a fibre blend comprising the steps of:

(i) providing a solution of a degradable polyester and a degradable copolymer, said copolymer comprising degradable ester units and from 1 to 50 % in number of phosphoester units versus the total number of monomer units in said copolymer, then
(ii) electrospinning said solution so as to obtain a fibre blend, then
(iii) optionally releasing negative charges of phosphoester units of the fibre blend, then
(iv) optionally dipping the fibre blend in a polysaccharide solution so as to obtain a coated fibre blend, then
(v) optionally dipping the coated fibre blend in a poly(phosphoester) solution and subsequently dipping the fibre blend in a polysaccharide solution so as to obtain a layered coated fibre blend, and then
(vi) optionally repeating step (v) until the desired layer number is reached.

**13.** A membrane comprising the fibre blend according to any of claims 1 to 11 wherein said membrane has a thickness of from 1 micrometer to 1 millimeter, preferably from 50 micrometers to 500 micrometers, more preferably from 100 micrometers to 300 micrometers.

**14.** The membrane according to the previous claim which is structured, preferably structured in a honeycomb pattern.

**15.** The membrane according to any of the claim 13 or 14 for use in a medical treatment.

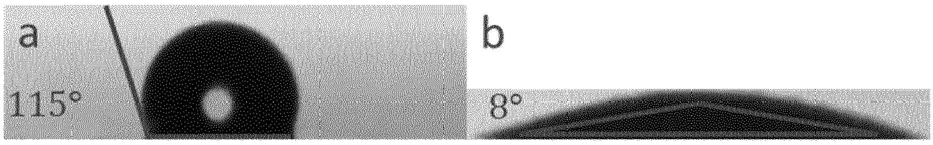


Figure 1

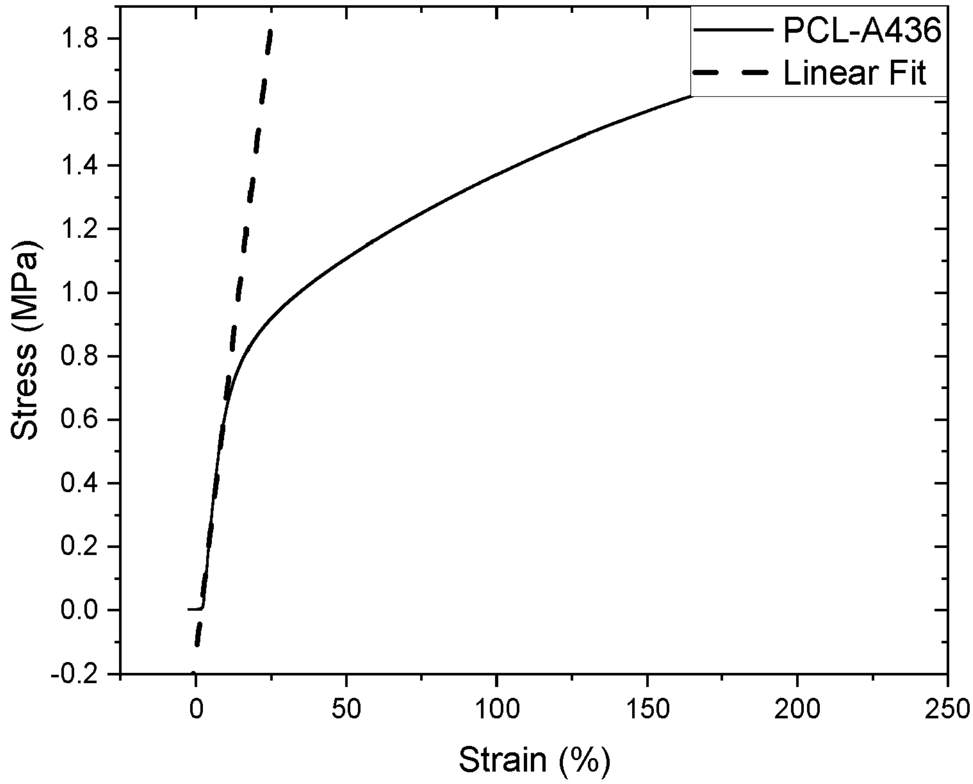


Figure 2A

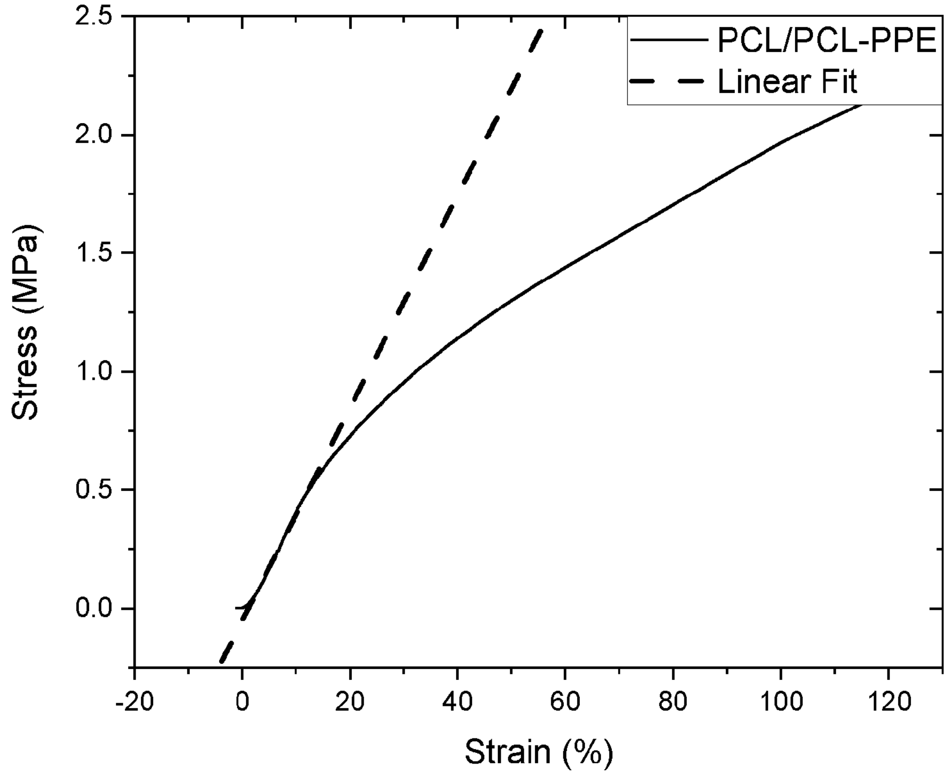


Figure 2B

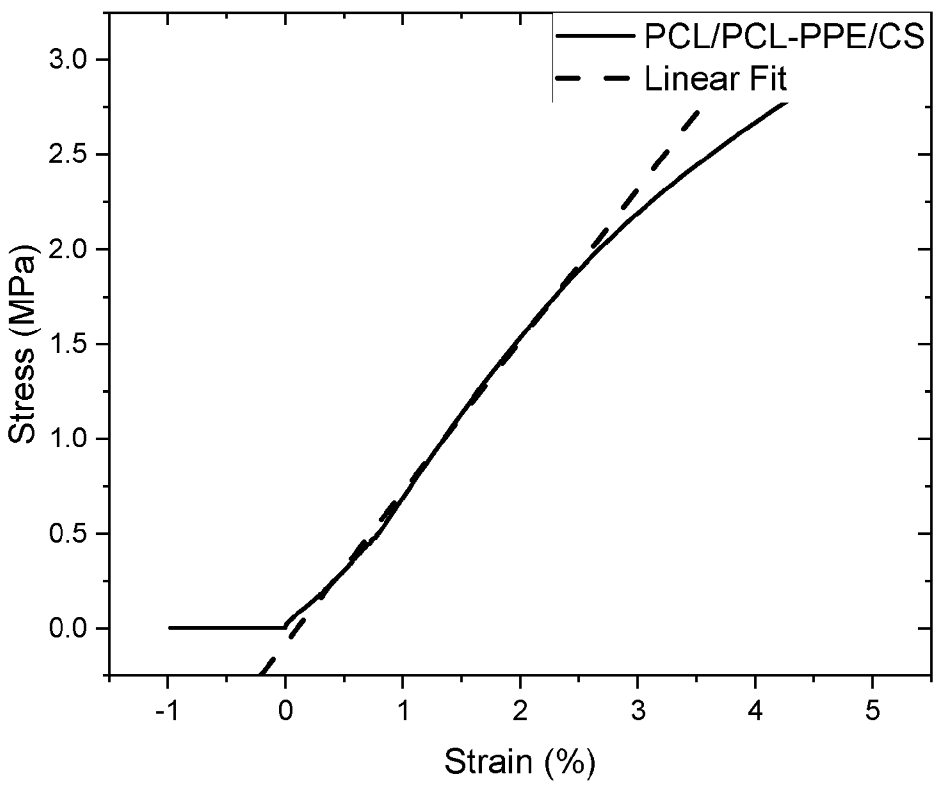


Figure 2C

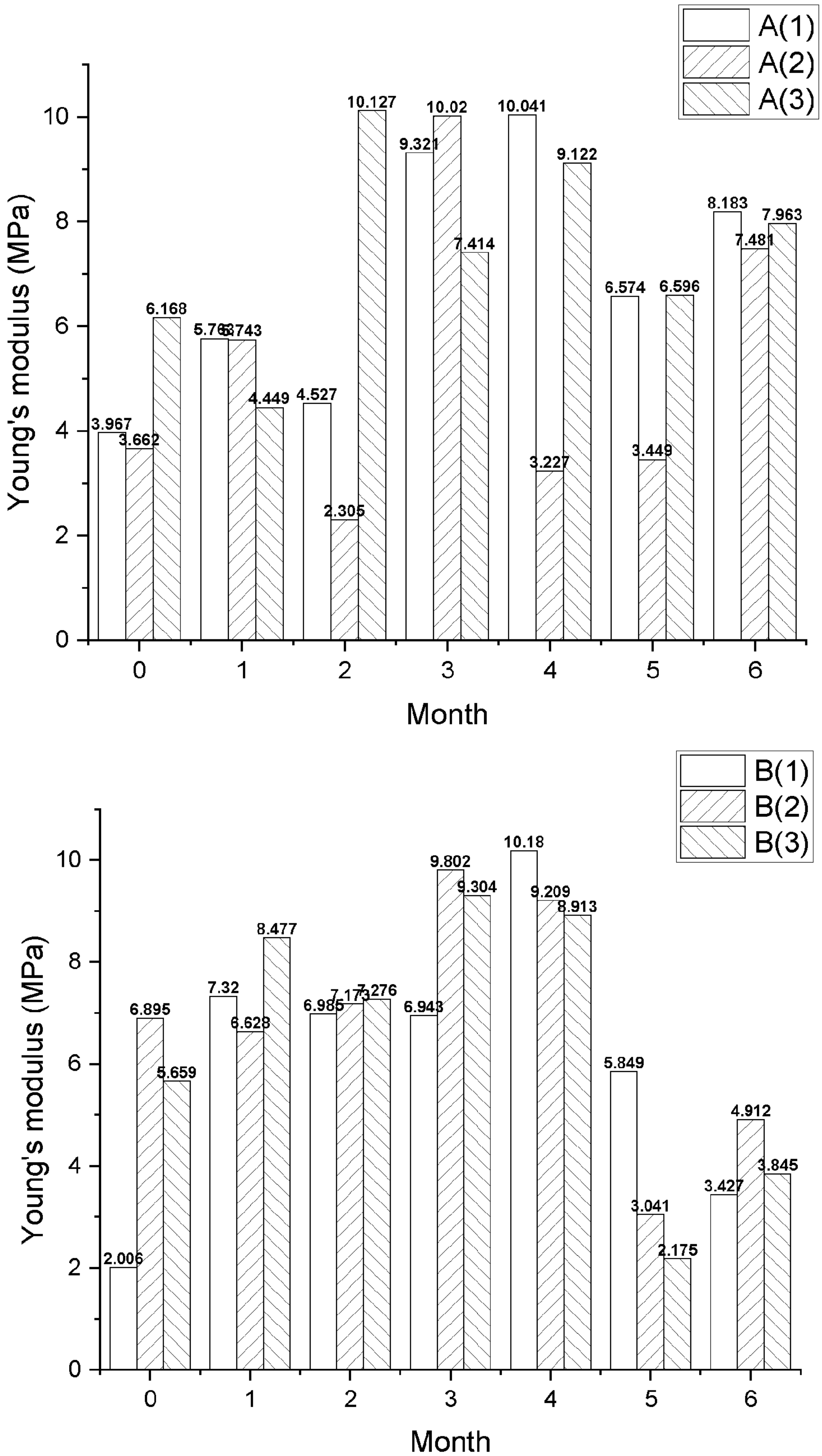
A(1)
A(2)
A(3)
Young's modulus (MPa)
Month
3.967
3.662
6.168
5.76
5.743
4.449
4.527
2.305
10.127
9.321
10.02
7.414
10.041
3.227
9.122
6.574
3.449
6.596
8.183
7.481
7.963
B(1)
B(2)
B(3)
Young's modulus (MPa)
Month
2.006
6.895
5.659
7.32
6.623
8.477
6.985
7.173
7.276
6.943
9.802
9.304
10.18
9.209
8.913
5.849
3.041
2.175
3.427
4.912
3.845

Figure 3

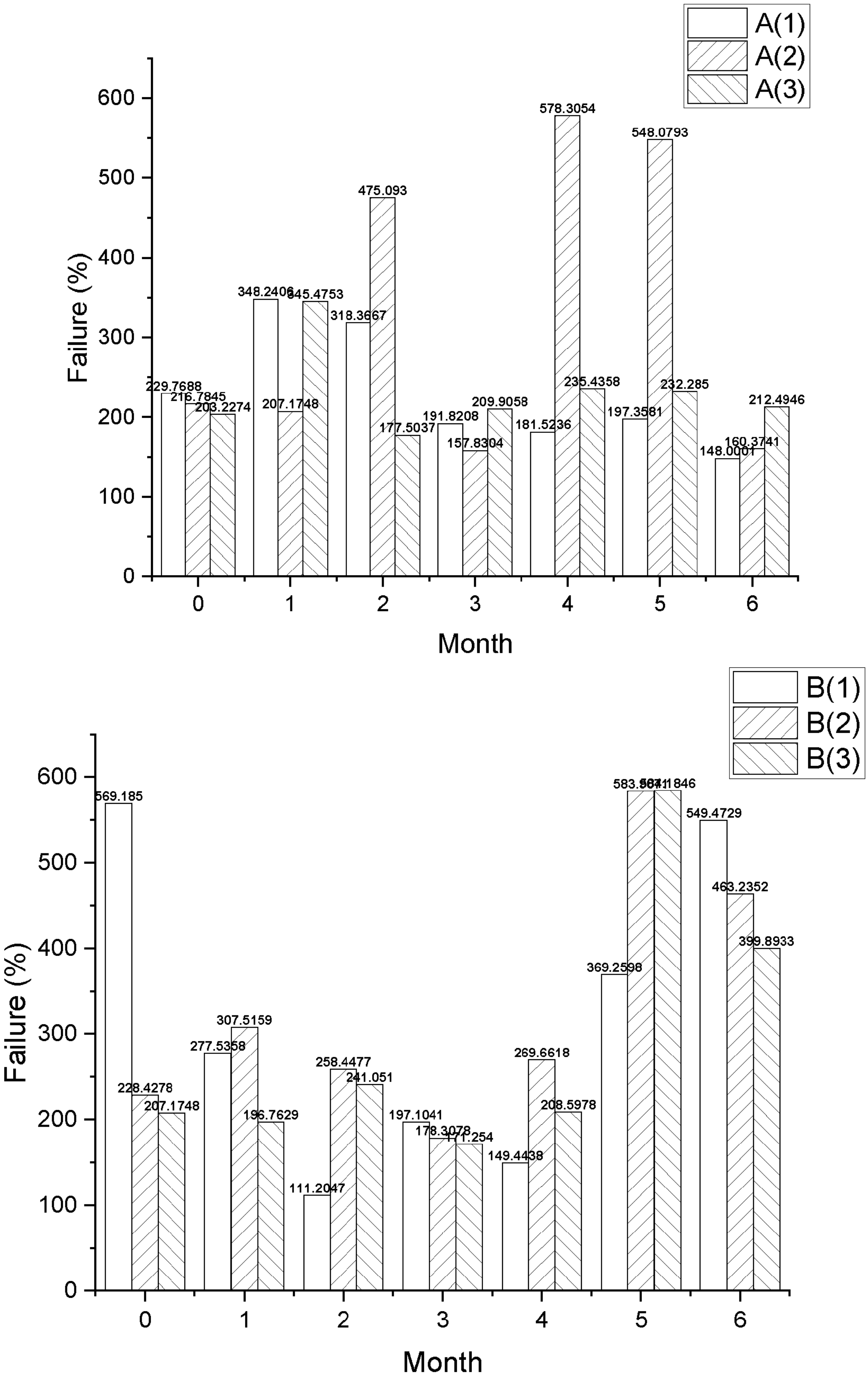
A(1)
A(2)
A(3)
Failure (%)
Month
0
100
200
300
400
500
600
0 1 2 3 4 5 6
229.7688
216.7845
203.2274
348.2406
207.1748
345.4753
318.3667
475.093
177.5037
191.8208
157.8304
209.9058
181.5236
578.3054
235.4358
197.3581
548.0793
232.285
148.0001
160.3741
212.4946
B(1)
B(2)
B(3)
569.185
228.4278
207.1748
277.5358
307.5159
196.7629
111.2047
258.4477
241.051
197.1041
178.3078
171.254
149.4438
269.6618
208.5978
369.2598
549.4729
463.2352
399.8933

Figure 4

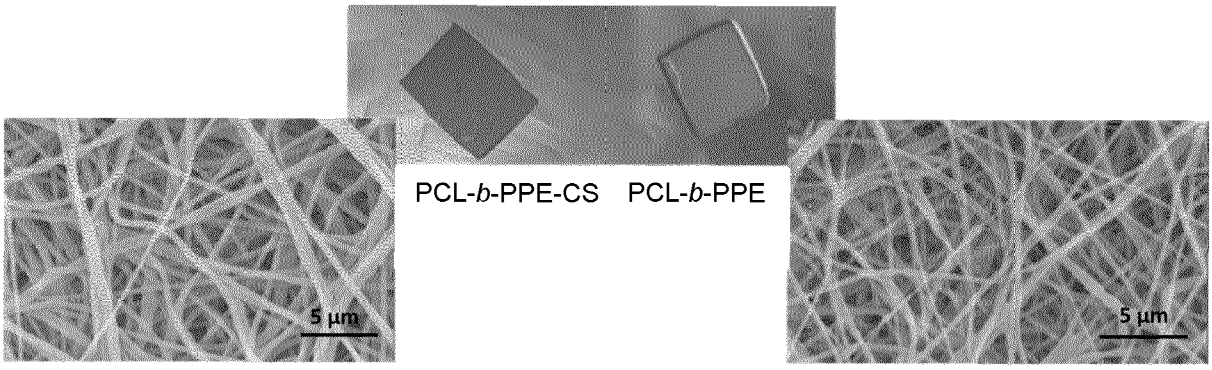


Figure 5

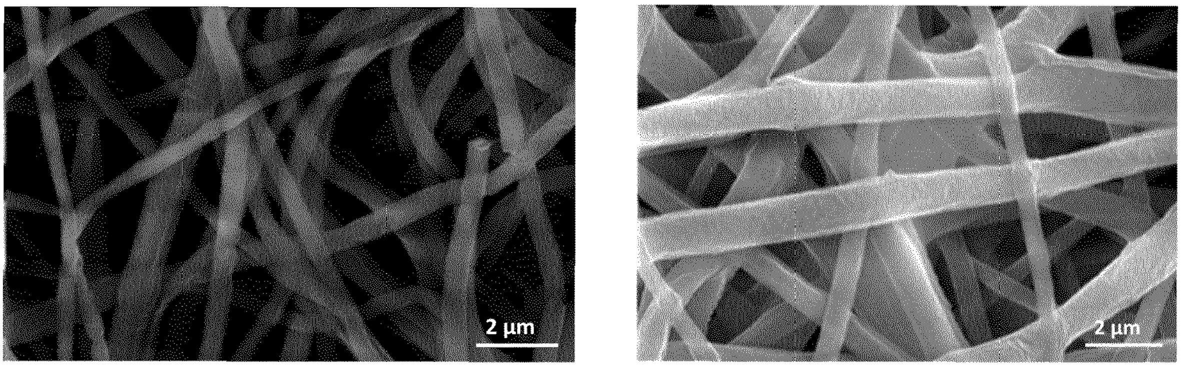


Figure 6

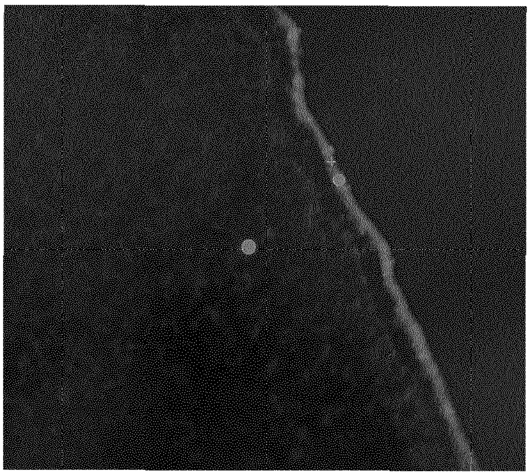

Figure 7

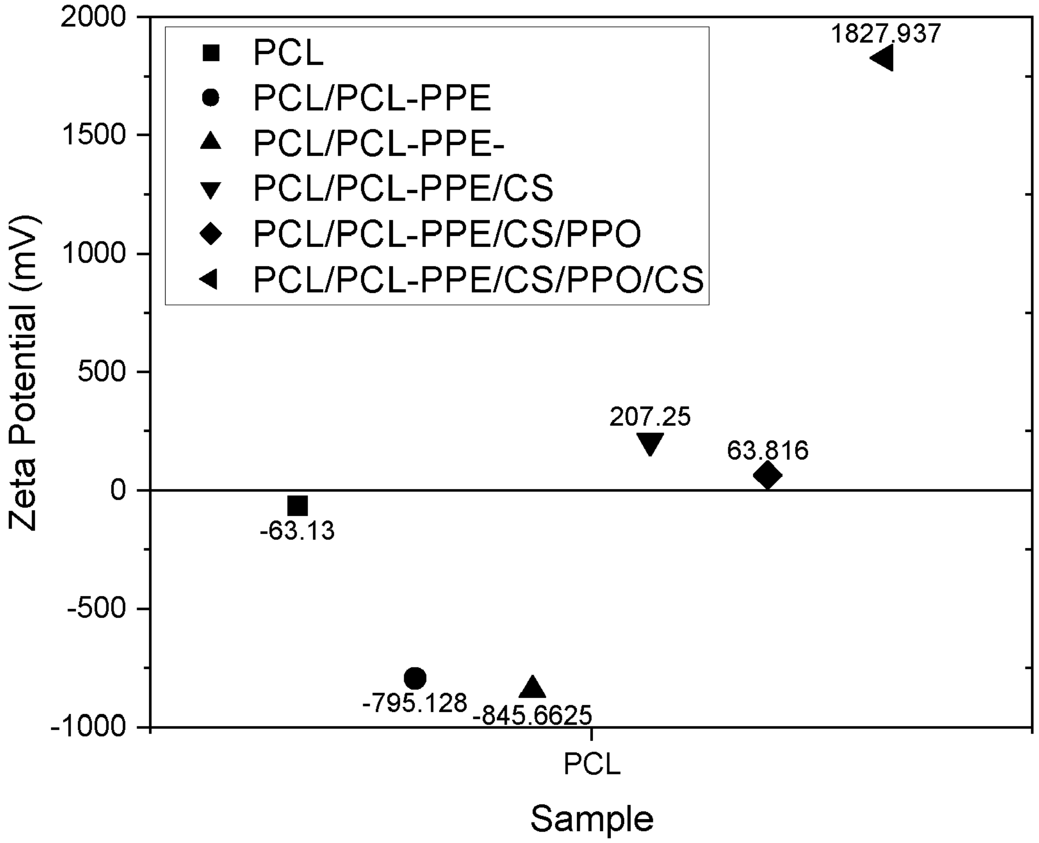
2000
1500
1000
500
0
-500
-1000
Zeta Potential (mV)
PCL
PCL/PCL-PPE
PCL/PCL-PPE-
PCL/PCL-PPE/CS
PCL/PCL-PPE/CS/PPO
PCL/PCL-PPE/CS/PPO/CS
1827.937
207.25
63.816
-63.13
-795.128
-845.6625
PCL
Sample

Figure 8

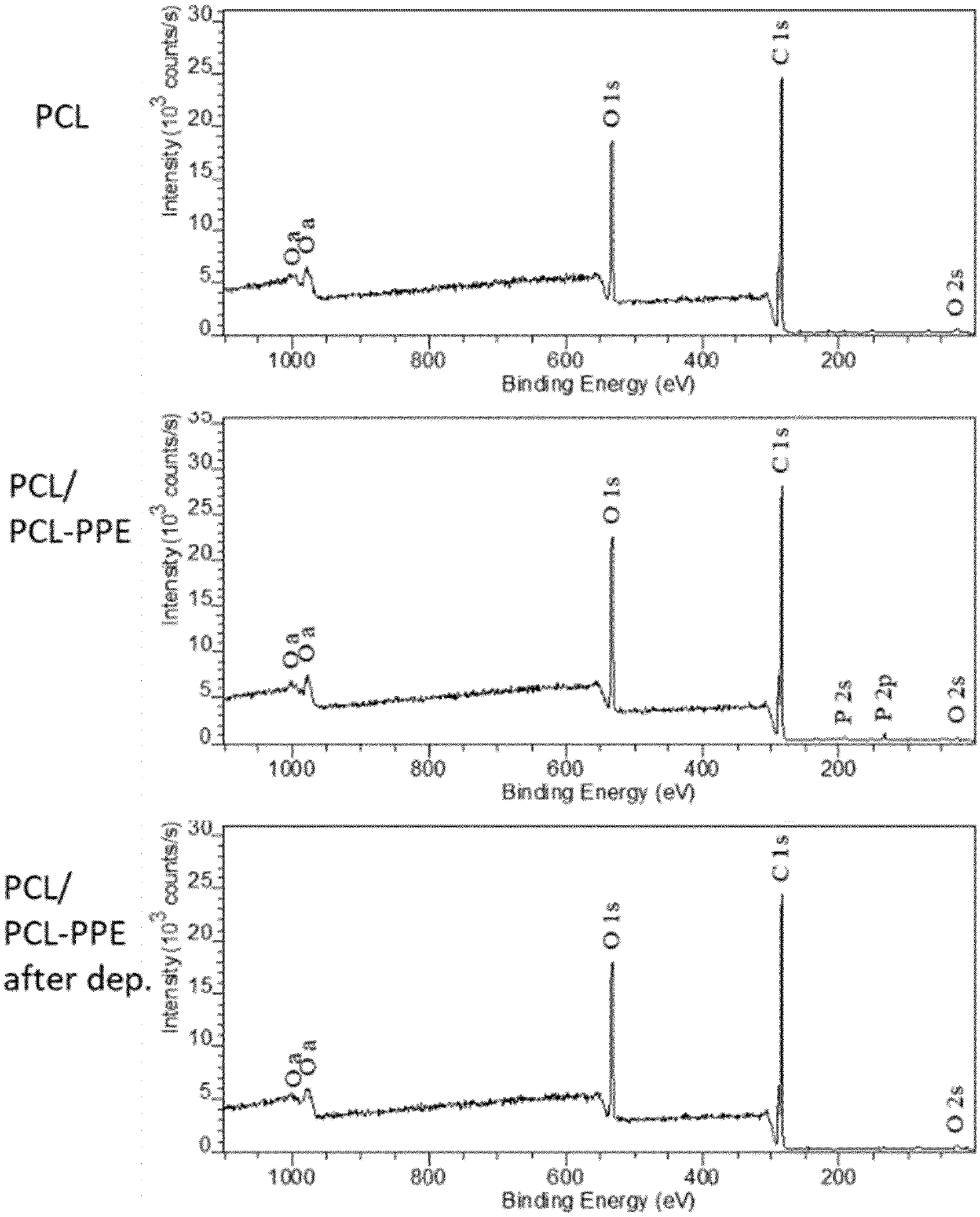
PCL
Intensity (10^3 counts/s)
Binding Energy (eV)
O a
O a
O 1s
C 1s
O 2s
PCL/
PCL-PPE
P 2s
P 2p
PCL/
PCL-PPE
after dep.

Figure 9

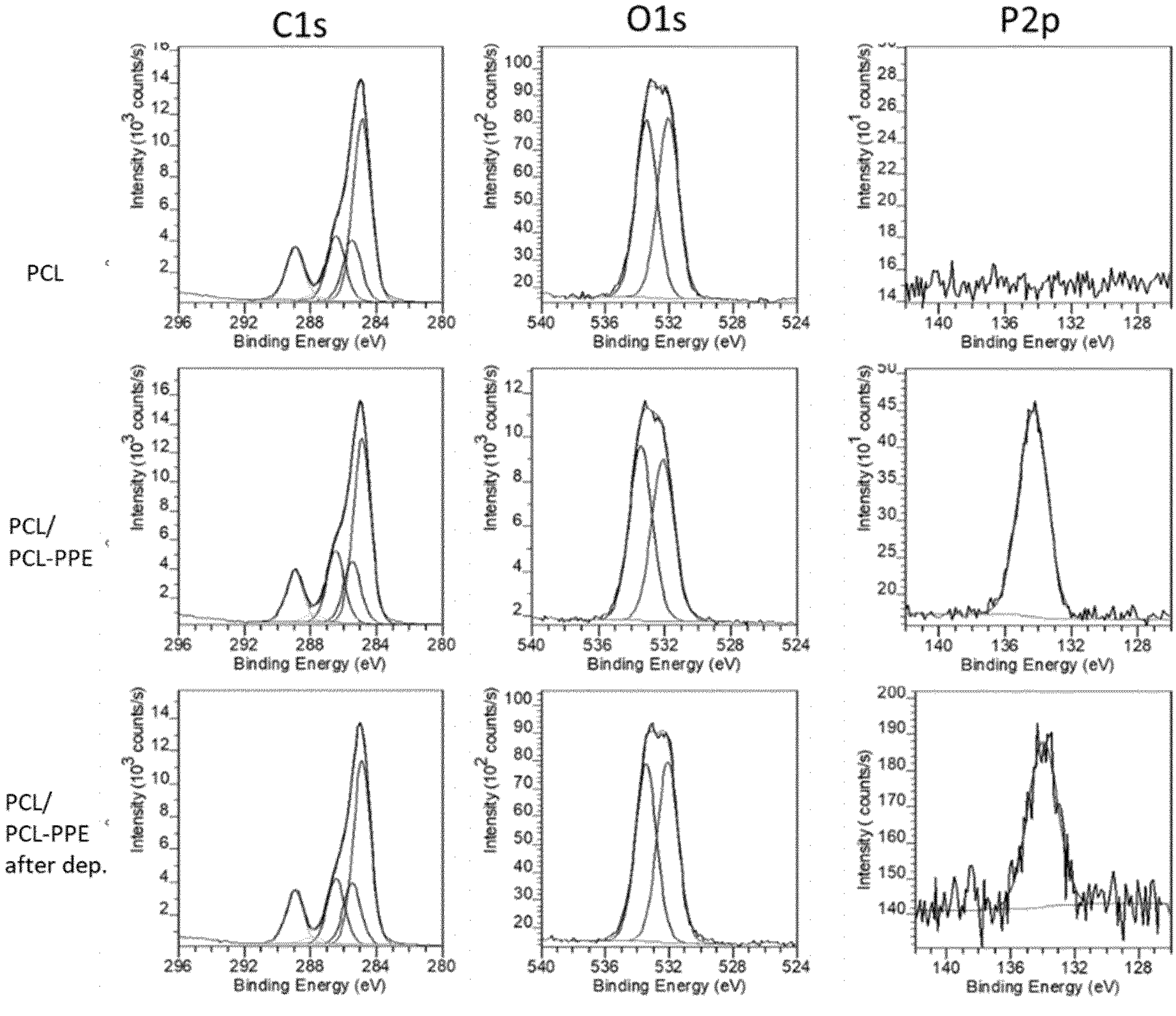
C1s
O1s
P2p
PCL
PCL/
PCL-PPE
PCL/
PCL-PPE
after dep.
Intensity ($10^3$ counts/s)
Intensity ($10^2$ counts/s)
Intensity ($10^1$ counts/s)
Intensity ( counts/s)
Binding Energy (eV)

Figure 10

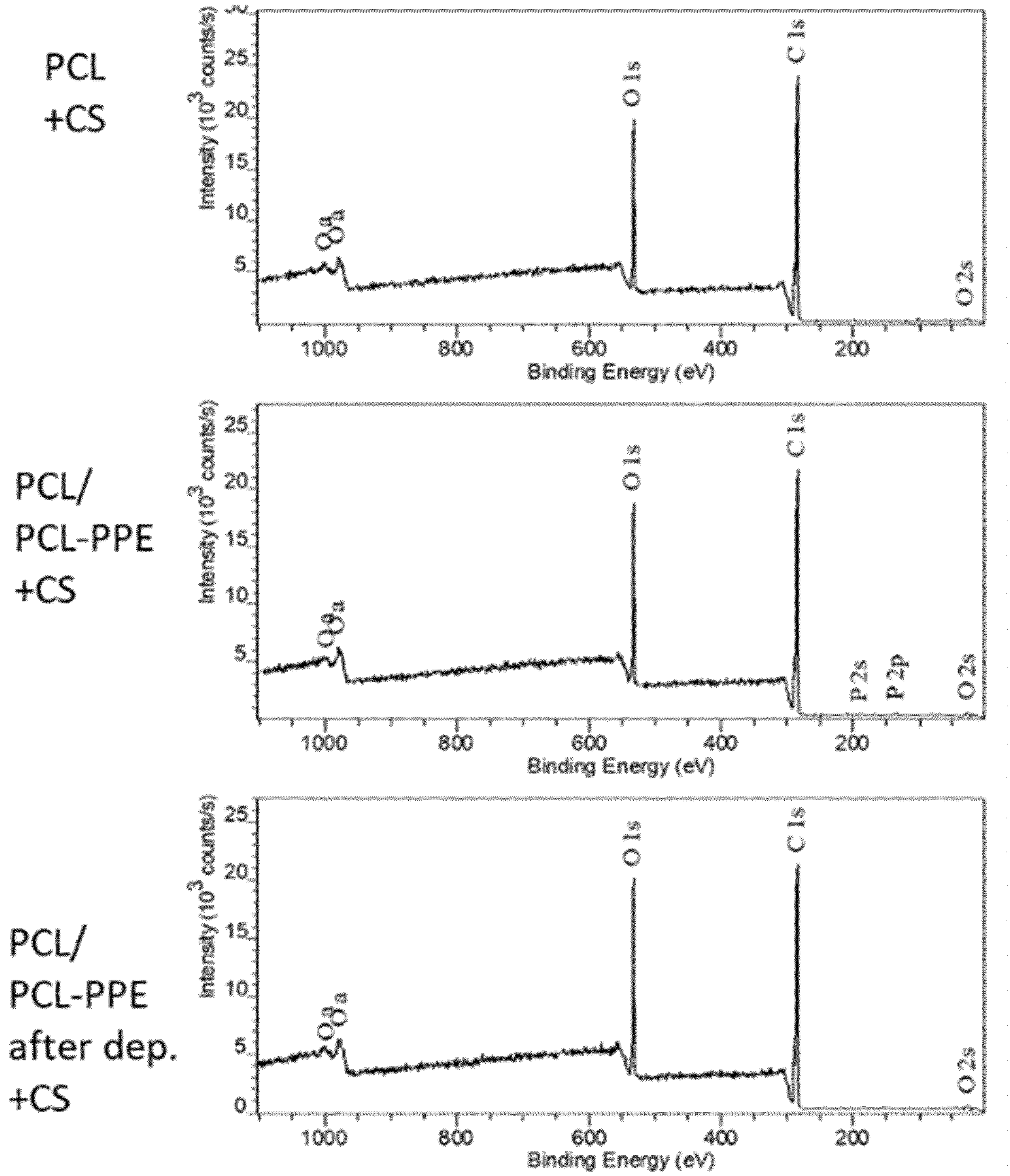
PCL
+CS
PCL/
PCL-PPE
+CS
PCL/
PCL-PPE
after dep.
+CS
Intensity (10^3 counts/s)
Binding Energy (eV)
O 1s
C 1s
O a
O 2s
P 2s
P 2p
1000
800
600
400
200

Figure 11

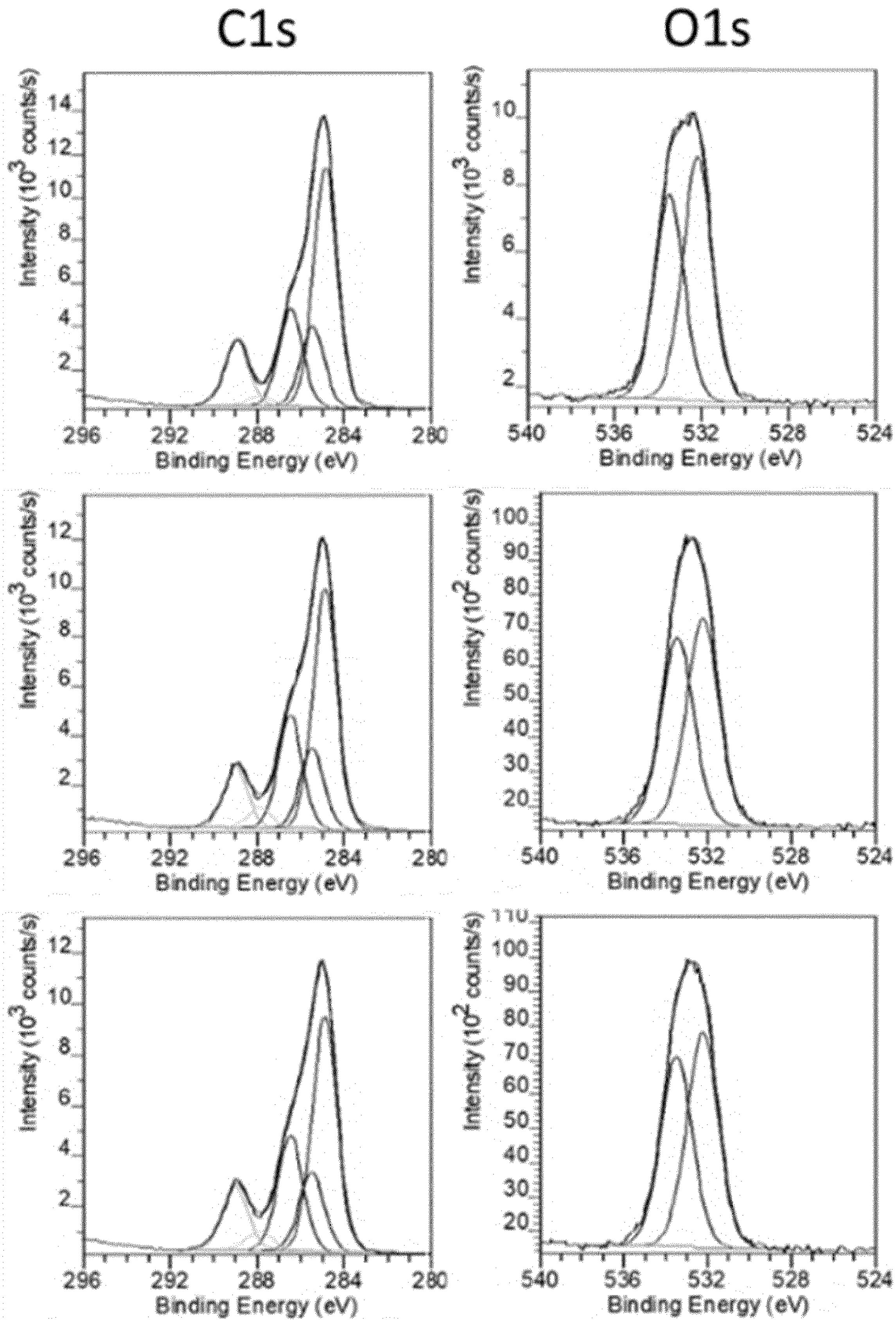
C1s
O1s
Intensity ($10^3$ counts/s)
Intensity ($10^2$ counts/s)
Binding Energy (eV)
296
292
288
284
280
540
536
532
528
524

Figure 12A

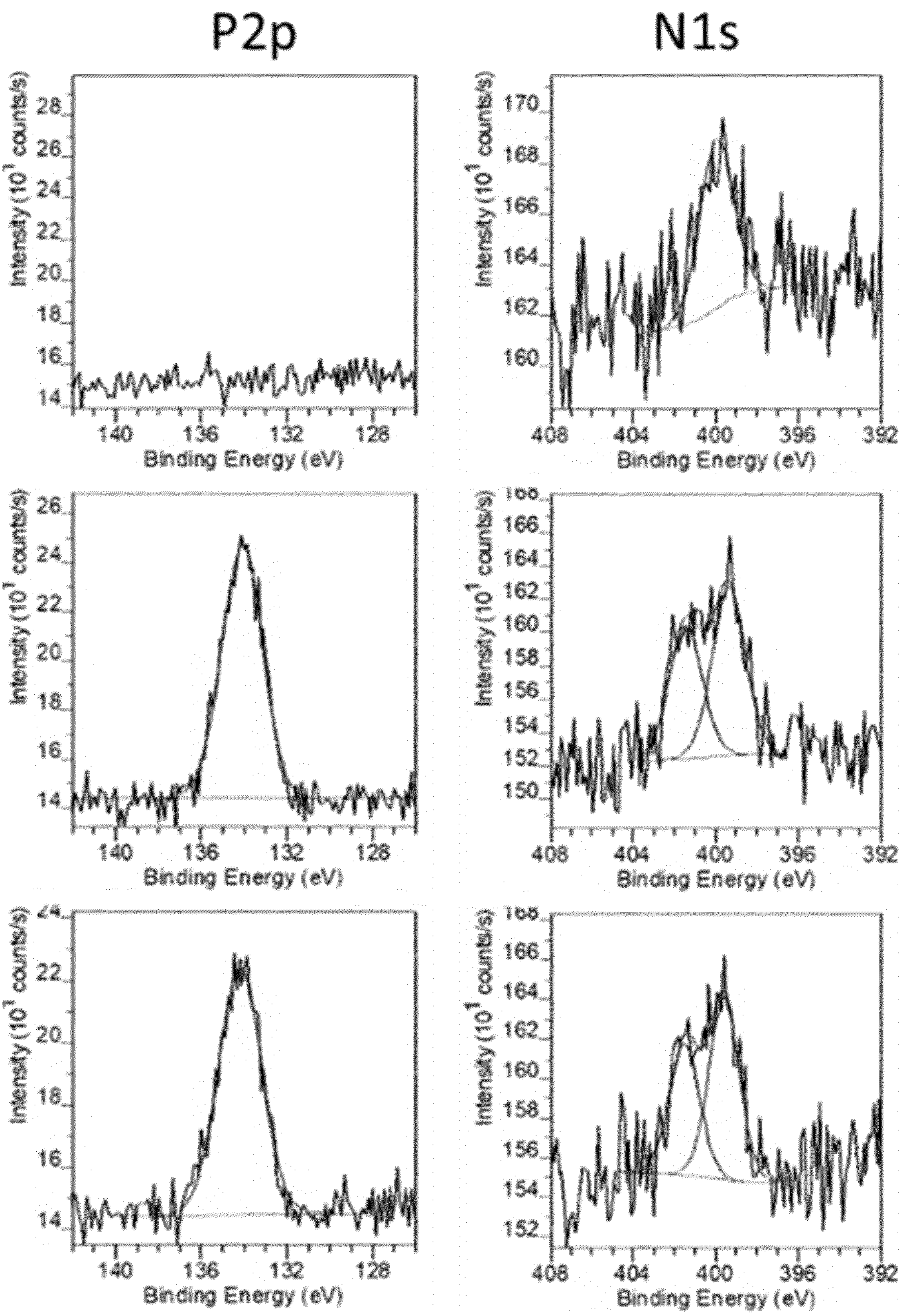
P2p
N1s
Intensity (10[1] counts/s)
Binding Energy (eV)
140
136
132
128
408
404
400
396
392

Figure 12B

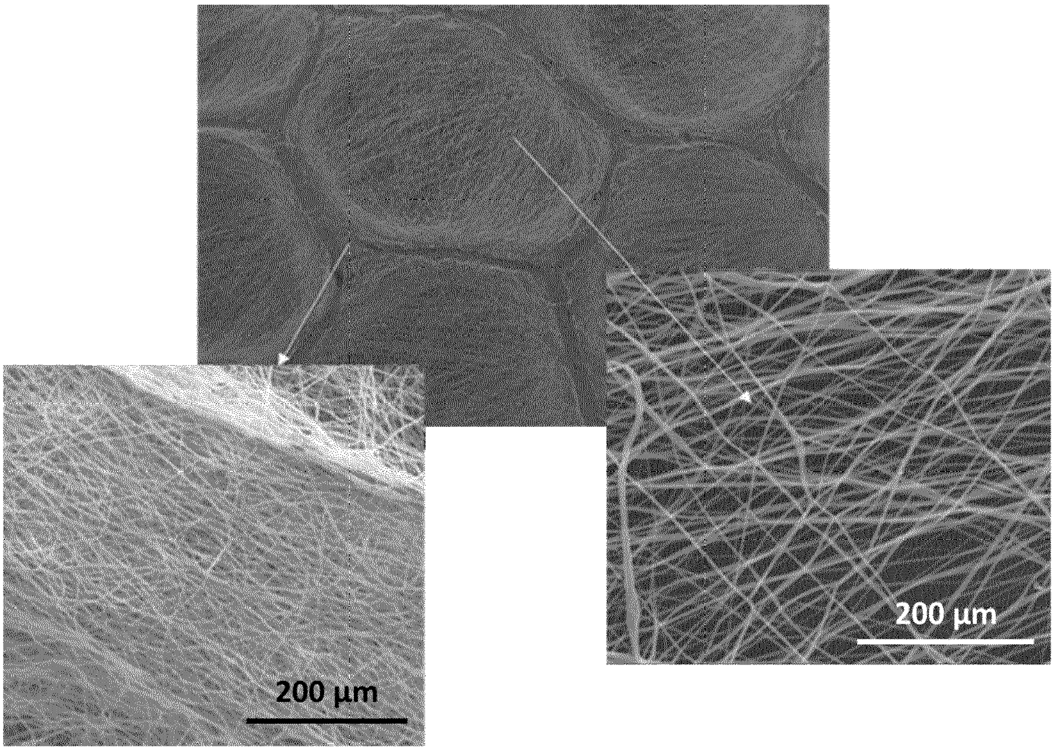


Figure 13

Europäisches Patentamt
European Patent Office
Office européen des brevets

# EUROPEAN SEARCH REPORT

Application Number
EP 24 16 5486

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | PADALHIN ANDREW R. ET AL: "Evaluation of the cytocompatibility hemocompatibility in vivo bone tissue regenerating capability of different PCL blends", JOURNAL OF BIOMATERIALS SCIENCE. POLYMER EDITION., vol. 25, no. 5, 22 January 2014 (2014-01-22), pages 487-503, XP093201613, NL ISSN: 0920-5063, DOI: 10.1080/09205063.2013.878870 * abstract * * page 489, paragraph 2.3 * ----- | 1-15 | INV. A61L27/56 A61L27/48 |
| A | LUO JINGJING ET AL: "Co-electrospun nano-/microfibrous composite scaffolds with structural and chemical gradients for bone tissue engineering", MATERIALS SCIENCE AND ENGINEERING C, vol. 119, 1 February 2021 (2021-02-01), page 111622, XP093201808, CH ISSN: 0928-4931, DOI: 10.1016/j.msec.2020.111622 * abstract * * page 3, paragraph 2.3; figure 1 * ----- | 1-15 | TECHNICAL FIELDS SEARCHED (IPC) A61L |
| A | WO 2022/271255 A1 (GEORGIA TECH RES INST [US]; JIANG CHEN [US] ET AL.) 29 December 2022 (2022-12-29) * claims 1, 3, 29 * ----- -/-- | 1-15 | |

The present search report has been drawn up for all claims

1

| Place of search | Date of completion of the search | Examiner |
|---|---|---|
| The Hague | 5 September 2024 | Guazzelli, Giuditta |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

page 1 of 2

Europäisches Patentamt
European Patent Office
Office européen des brevets

# EUROPEAN SEARCH REPORT

Application Number
EP 24 16 5486

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | SING YIAN CHEW ET AL: "Sustained Release of Proteins from Electrospun Biodegradable Fibers", BIOMACROMOLECULES, vol. 6, no. 4, 1 July 2005 (2005-07-01), pages 2017-2024, XP055172488, ISSN: 1525-7797, DOI: 10.1021/bm0501149 * abstract * ----- | 1-15 | |
| | | | TECHNICAL FIELDS SEARCHED (IPC) |

The present search report has been drawn up for all claims

1

| Place of search | Date of completion of the search | Examiner |
|---|---|---|
| The Hague | 5 September 2024 | Guazzelli, Giuditta |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
.......................................................................................................
& : member of the same patent family, corresponding document

EPO FORM 1503 03.82 (P04C01)

page 2 of 2

# ANNEX TO THE EUROPEAN SEARCH REPORT ON EUROPEAN PATENT APPLICATION NO.

EP 24 16 5486

This annex lists the patent family members relating to the patent documents cited in the above-mentioned European search report. The members are as contained in the European Patent Office EDP file on
The European Patent Office is in no way liable for these particulars which are merely given for the purpose of information.

05-09-2024

| Patent document cited in search report | Publication date | Patent family member(s) | Publication date |
|---|---|---|---|
| WO 2022271255 A1 | 29-12-2022 | NONE | |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description


- *Macromol. Biosci.*, 2016, vol. 16, 1745-1761
- **CLEMENT et al.** *Macromolecule*, 2012, vol. 45, 4476-4486